# EUROPEAN PATENT SPECIFICATION

(11) **EP 2 380 516 B1**
(45) Date of publication and mention of the grant of the patent: **09.03.2016**
(21) Application number: 09838874.7
(22) Date of filing: 22.12.2009
(51) Int. Cl.: A61B 18/12

(54) **TREATMENT DEVICE AND TREATMENT TOOL**
BEHANDLUNGSVORRICHTUNG UND BEHANDLUNGSWERKZEUG
DISPOSITIF DE TRAITEMENT ET OUTIL DE TRAITEMENT

(30) Priority: 21.01.2009 US 356790
(43) Date of publication of application: 26.10.2011
(73) Proprietor: Olympus Corporation, Shibuya-ku Tokyo (JP)
(72) Inventor: SAKAO, Satomi, Tokyo 192-8512 (JP); IIDA, Koji, Tokyo 192-8512 (JP)
(74) Representative: Schicker, Silvia
(86) International application number: PCT/JP2009/071276
(87) International publication number: WO 2010/084684

(56) References cited:
- JP-A- 2008 212 679
- JP-A- 2008 212 679
- JP-T- 2005 534 451
- JP-T- 2005 534 451
- US-A1- 2007 043 297
- US-A1- 2008 319 442

## Description

### Technical Field

This disclosure relates to a medical treatment apparatus and a medical treatment instrument that are capable of joining a plurality of living tissues together using energy. The invention is set out in the appended claims.

### Background Art

In surgical operations including an abdominal operation and a laparoscopic operation, a tubular tissue or organ of, for example, a blood vessel may be sealed, or other tissues may be joined together. For example, a suture or clip is used to seal the blood vessel to be disjoined. A suture or staple is used to seal or anastomose cut ends of a digestive tract. In addition, techniques using energy have been in use recently. A high-frequency device or ultrasonic device is constantly used to seal a blood vessel, and moreover, other devices used for thicker living tissues are also making progress. In such a procedure, living tissues are held with a forceps-shaped device and treated. Energy is input to the held living tissues from an electrode disposed on the surface of a holding member and from an ultrasonic probe having a holding function together, such that the living tissues are joined together. In such a procedure, macromolecules of the living tissues are denatured, so that the living tissues themselves can be used as adhesive components to join the living tissues together.

Among the macromolecules of the living tissues, collagen is one of the components that are most easily bonded. The living tissues can be firmly joined together if collagens in the joint surfaces of the living tissues can be bonded together. This enables a stable treatment.

However, since the surface of an organ is covered with components other than collagen such as epithelial cells, collagens in the joint surfaces of the living tissues can not be joined together merely by holding the tissues and outputting energy thereto. In order to enable the joint surfaces of the living tissues to be joined together by collagens, it is necessary to remove the components other than collagen on the surface of an organ, in particular, the epithelial cells.

Techniques for removing surface tissues include, for example, cavitation using ultrasonic energy, transpiration of living tissues caused by high-frequency energy, and physical friction. For example, the techniques for removing living tissues by ultrasonic energy are disclosed in EP 1 526 825 A1 and USP 6,736,814 B2. EP 1 526 825 A1 describes a technique which uses ultrasonic vibrations to perform a procedure called debridment for eliminating damaged living tissues. USP 6,736,814 B2 describes a technique which uses ultrasonic suction when treating the central nerve system. There is also a technique described in USP 6,461,350 B1 which uses high-frequency energy to remove living tissues. The technique described in USP 6,736,814 B2 uses high-frequency energy to remove adipose cells under epidermal tissues. On the other hand, techniques which have both ultrasonic and high-frequency devices between forceps structures for holding and joining living tissues include USP 6,500,176 B1, Jpn. Pat. Appln. KOKAI Publication No. 2007-229270, and USP 6, 736, 814B2.

However, the objects of the prior art described above are to remove living tissues themselves. The prior art documents do not involve a technique of removing surfaces of living tissues to bond them.

For example, the objects of the prior art disclosed in EP 1 526 825 A1 and USP 6,736,814 B2 are to remove living tissues. In the prior art, exposure of parts of living tissues necessary to join the living tissues is not taken into consideration. Therefore, the prior art does not comprise a structure of a grasping portion necessary to join living tissues.

Further, both USP 6,500,176 B1 and Jpn. Pat. Appln. KOKAI Publication No. 2007-229270 disclose techniques in which an ultrasonic probe and a high-frequency electrode are provided between forceps structures for holding and joining living tissues. However, in the inventions disclosed in these prior art publications, energy applying means is provided between forceps structures merely for the purpose of efficiently applying energy to living tissues. These publications do not disclose a technique for removing macromolecules of surface tissues of an organ to be bonded together and exposing a component which can be easily bonded.

USP 6,736,814 B2 also discloses a technique in which an ultrasonic probe is provided between forceps structures with an electrode for holding and joining living tissues. The ultrasonic probe is used only to remove living tissues of a brain or the like, while the grasping portion is used to stop the bleeding when removing such a tissue. In other words, the ultrasonic probe is not used to remove materials from the joint surfaces of the target living tissues to be joined. Moreover, the apparatus or instrument of this prior art is not designed to treat the joint surfaces of two living tissues.

US 2008/0319442 A1 discloses several embodiments of vessel sealing cutting assemblies (blade assemblies), and, in particular, a "guillotine-like" instrument comprising an ultrasonically energized blade for cutting tissue after seuling.

### Disclosure of Invention

An object of the present disclosure to provide a medical treatment apparatus and a medical treatment instrument configured to expose collagen that has strong adhesive power on surfaces of living tissues by breaking and removing macromolecules, which have weak bonding power, when the living tissues are to be joined together.

According to a first aspect of the present disclosure there is provided a medical treatment apparatus to join target living tissues in a body, the medical treatment apparatus including: an energy source which applies energy to the target living tissues; a first treating portion which joins the target living tissues together when energy is applied thereto from the energy source; a second treating portion which is interposed between the target living tissues and which removes surface portions of tissues in the joint surfaces of the target living tissues; an operation portion; and a controller. The first treating portion includes at least a pair of holding members having holding surfaces to hold the target living tissues, and energy emitters which are provided on the holding surfaces of the holding members and which join the target living tissues together when energy is applied thereto from the energy source. The operation portion has a function of operating the holding members so that at least one of the holding members moves relative to the other. The controller controls outputs from the energy emitters.

According to a second aspect of the present disclosure there is provided a medical treatment instrument to join target living tissues in a body, the treatment instrument including: a first treating portion which joins the target living tissues together when energy is applied thereto from an energy source; a second treating portion which is interposed between the target living tissues and which removes surface portions of tissues in the joint surfaces of the target living tissues; and an operation portion. The first treating portion includes at least a pair of holding members having holding surfaces to hold the target living tissues, and energy emitters which are provided on the holding surfaces of the holding members and which join the target living tissues together when energy is applied thereto from the energy source. The operation portion has a function of operating the holding members so that at least one of the holding members moves relative to the other.

### Brief Description of Drawings

FIG. 1A is a schematic perspective view showing a medical treatment apparatus according to a first embodiment;
FIG. 1B is a partial sectional view of a handle and a shaft of an energy treatment instrument of the medical treatment apparatus according to the first embodiment;
FIG. 2 is a schematic diagram showing the medical treatment apparatus according to the first embodiment;
FIG. 3A is a schematic longitudinal sectional view showing the shaft and a treatment portion in which first and second holding members are closed and in which an ultrasonic probe is disposed between the first and second holding members of the energy treatment instrument of the medical treatment apparatus according to the first embodiment;
FIG. 3B is a schematic longitudinal sectional view showing the shaft and the treatment portion in which the first and second holding members are opened and in which the ultrasonic probe is disposed between the first and second holding members of the energy treatment instrument of the medical treatment apparatus according to the first embodiment;
FIG. 3C is a schematic longitudinal sectional view showing the shaft and the treatment portion in which the first and second holding members are opened and in which the ultrasonic probe is drawn into the shaft from between the first and second holding members of the energy treatment instrument of the medical treatment apparatus according to the first embodiment;
FIG. 3D is a schematic cross sectional view along the line 3D-3D in FIG. 3A, wherein the first and second holding members of the treatment portion are closed, and the ultrasonic probe is disposed between the first and second holding members of the energy treatment instrument of the medical treatment apparatus according to the first embodiment;
FIG. 4A is a schematic view showing a holding surface of the main body of the first holding member of the treatment portion of the energy treatment instrument of the medical treatment apparatus according to the first embodiment;
FIG. 4B is a schematic cross sectional view along the line 4B-4B in FIG. 4A, showing the main body of the first holding member of the treatment portion of the energy treatment instrument of the medical treatment apparatus according to the first embodiment;
FIG. 5 is a flowchart for joining living tissues by use of the medical treatment apparatus according to the first embodiment;
FIG. 6 is a schematic graph showing the relation of the impedance of living tissues with time when the medical treatment apparatus is used to continuously apply high-frequency energy to the living tissues and thereby treat the living tissues;
FIG. 7A is a schematic view showing the holding surface of the main body of the first holding member of the treatment portion of the energy treatment instrument of the medical treatment apparatus according to a modification of the first embodiment;
FIG. 7B is a schematic cross sectional view along the line 7B-7B in FIG. 7A, showing the main body of the first holding member of the treatment portion of the energy treatment instrument of the medical treatment apparatus according to the modification of the first embodiment;
FIG. 8 is a schematic perspective view showing the medical treatment apparatus according to a modification of the first embodiment;
FIG. 9 is a schematic perspective view showing the medical treatment apparatus according to a modification of the first embodiment;
FIG. 10A is a schematic diagram showing how to treat by a bipolar medical treatment apparatus according to the first embodiment;
FIG. 10B is a schematic diagram showing how to treat by a monopolar medical treatment apparatus according to the first embodiment;
FIG. 11 is a schematic perspective view showing a medical treatment apparatus according to a second embodiment;
FIG. 12 is a schematic diagram showing the medical treatment apparatus according to the second embodiment;
FIG. 13A is a schematic longitudinal sectional view showing a shaft and a treatment portion in which first and second holding members are closed and in which an ultrasonic suction probe is disposed between the first and second holding members of an energy treatment instrument of the medical treatment apparatus according to the second embodiment;
FIG. 13B is a schematic longitudinal sectional view showing the shaft and the treatment portion in which the first and second holding members are opened and in which the ultrasonic suction probe is disposed between the first and second holding members of the energy treatment instrument of the medical treatment apparatus according to the second embodiment;
FIG. 13C is a schematic longitudinal sectional view showing the shaft and the treatment portion in which the first and second holding members are opened and in which the ultrasonic suction probe is drawn into the shaft from between the first and second holding members of the energy treatment instrument of the medical treatment apparatus according to the second embodiment;
FIG. 13D is a schematic cross sectional view along the line 13D-13D in FIG. 13A, wherein the first and second holding members of the treatment portion are closed, and the ultrasonic suction probe is disposed between the first and second holding members of the energy treatment instrument of the medical treatment apparatus according to the second embodiment;
FIG. 14 a flowchart for joining living tissues by use of the medical treatment apparatus according to the second embodiment;
FIG. 15A is a schematic view showing a holding surface of a main body of the first holding member of the treatment portion of the energy treatment instrument of the medical treatment apparatus according to a modification of the second embodiment;
FIG. 15B is a schematic longitudinal sectional view along the line 15B-15B in FIG. 15A, showing the main body and a base of the first holding member of the treatment portion of the energy treatment instrument of the medical treatment apparatus according to the modification of the second embodiment;
FIG. 15C is a schematic longitudinal sectional view along the line 15C-15C in FIG. 15A, showing the main body and a base of the first holding member of the treatment portion of the energy treatment instrument of the medical treatment apparatus according to the modification of the second embodiment;
FIG. 16 is a schematic diagram showing a medical treatment apparatus according to a third embodiment;
FIG. 17A is a schematic longitudinal sectional view showing a shaft and a treatment portion in which first and second holding members are closed and in which a rod electrode is disposed between the first and second holding members of an energy treatment instrument of the medical treatment apparatus according to the third embodiment;
FIG. 17B is a schematic longitudinal sectional view showing the shaft and the treatment portion in which the first and second holding members are opened and in which the rod electrode is disposed between the first and second holding members of the energy treatment instrument of the medical treatment apparatus according to the third embodiment;
FIG. 17C is a schematic longitudinal sectional view showing the shaft and the treatment portion in which the first and second holding members are opened and in which the rod electrode is drawn into the shaft from between the first and second holding members of the energy treatment instrument of the medical treatment apparatus according to the third embodiment;
FIG. 17D is a schematic cross sectional view along the line 17D-17D in FIG. 17A, wherein the first and second holding members of the treatment portion are closed, and the rod electrode is disposed between the first and second holding members of the energy treatment instrument of the medical treatment apparatus according to the third embodiment;
FIG. 18 a flowchart for joining living tissues by use of the medical treatment apparatus according to the third embodiment;
FIG. 19A is a schematic diagram showing how to treat by a bipolar medical treatment apparatus according to the third embodiment;
FIG. 19B is a schematic diagram showing how to treat by a monopolar medical treatment apparatus according to the third embodiment;
FIG. 20 is a schematic partial sectional view of a medical treatment apparatus according to a fourth embodiment;
FIG. 21 is a schematic diagram showing the medical treatment apparatus according to the fourth embodiment;
FIG. 22A is a schematic longitudinal sectional view showing a shaft and a treatment portion in which first and second holding members are closed and in which a detachment member is disposed between the first and second holding members of an energy treatment instrument of the medical treatment apparatus according to the fourth embodiment;
FIG. 22B is a schematic longitudinal sectional view showing the shaft and the treatment portion in which the first and second holding members are opened and in which the detachment member is disposed between the first and second holding members of the energy treatment instrument of the medical treatment apparatus according to the fourth embodiment;
FIG. 22C is a schematic longitudinal sectional view showing the shaft and the treatment portion in which the first and second holding members are opened and in which the detachment member is drawn into the shaft from between the first and second holding members of the energy treatment instrument of the medical treatment apparatus according to the fourth embodiment;
FIG. 22D is a schematic cross sectional view along the line 22D-22D in FIG. 22A, wherein the first and second holding members of the treatment portion are closed, and the detachment member is disposed between the first and second holding members of the energy treatment instrument of the medical treatment apparatus according to the fourth embodiment; and
FIG. 23 a flowchart for joining living tissues by use of the medical treatment apparatus according to the fourth embodiment. The invention is set out in the appended claims. The embodiments of the present description which do not fall within the scope of said claims are provided for illustrative purposes only and do not form part of the present invention.

### [First Embodiment]

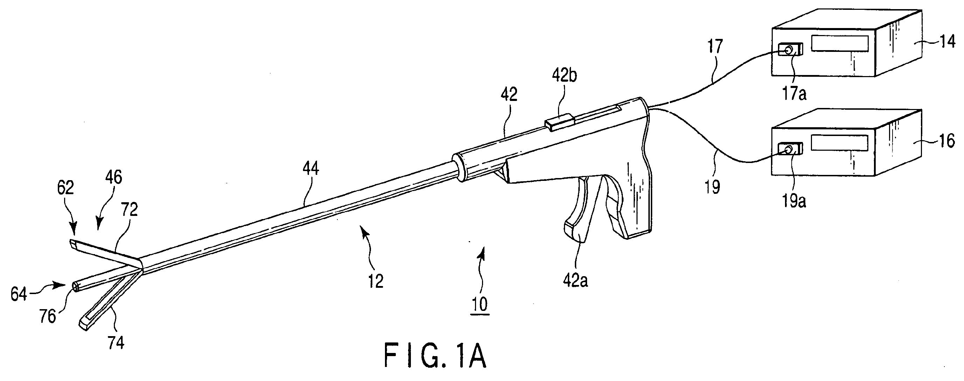

A first embodiment is described with FIG. 1A to FIG. 10B.

Here, a linear bipolar high-frequency energy treatment instrument 12 for a treatment, for example, through an abdominal wall is described as an example of an energy treatment instrument.

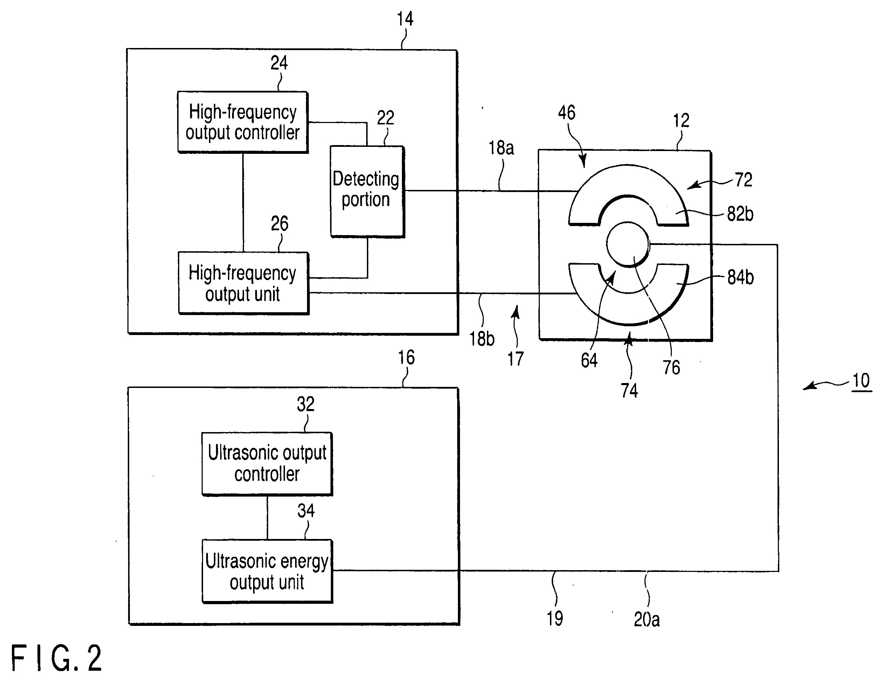

As shown in FIG. 1A and FIG. 2, a medical treatment apparatus 10 includes the energy treatment instrument (medical treatment instrument) 12, a high-frequency energy source 14 for providing high-frequency energy to the energy treatment instrument 12, and an ultrasonic energy source 16 for providing ultrasonic energy to the energy treatment instrument 12. The medical treatment apparatus 10 is connected to the high-frequency energy source 14 by a connector 17a of a cable 17 extending from the energy treatment instrument 12. The medical treatment apparatus 10 is connected to the ultrasonic energy source 16 by a connector 19a of a cable 19 extending from the energy treatment instrument 12.

As shown in FIG. 2, the high-frequency energy source 14 includes a detecting portion 22, a high-frequency energy controller (hereinafter referred to as a high-frequency output controller) 24, and a high-frequency energy output unit (hereinafter referred to as a high-frequency output unit) 26. The detecting portion 22 is connected to a later-described high-frequency electrode 82b of the energy treatment instrument 12. The high-frequency output controller 24 and the high-frequency output unit 26 of the high-frequency energy source 14 are connected to the detecting portion 22. The high-frequency output controller 24 is further connected to the high-frequency output unit 26. Moreover, the high-frequency output unit 26 is connected to the high-frequency electrode 82b of a later-described first holding member 72 of the energy treatment instrument 12 through the detecting portion 22, and is also connected to a high-frequency electrode 84b of a later-described second holding member 74 of the energy treatment instrument 12.

The detecting portion 22 detects electric biological information regarding living tissues held by the later-described first and second holding members (a pair of holding members) 72, 74 of the energy treatment instrument 12. That is, the detecting portion 22 detects the values of a current and a voltage flowing through the living tissues held between the first and second holding members 72, 74, calculates the value of the impedance Z from the detected current and voltage values, and provides the calculated impedance Z as biological information. The high-frequency output unit 26 outputs high-frequency energy under the control of the high-frequency output controller 24. Thus, the high-frequency output controller 24 can control the output of the high-frequency energy from the high-frequency output unit 26 to the energy treatment instrument 12 on the basis of the biological information detected by the detecting portion 22.

In addition, an unshown foot switch or hand switch is connected to the high-frequency energy source 14.

The ultrasonic energy source 16 includes an ultrasonic energy controller (hereinafter referred to as an ultrasonic output controller) 32, and an ultrasonic energy output unit (hereinafter referred to as an ultrasonic energy output unit) 34. The ultrasonic output controller 32 is connected to the ultrasonic energy output unit 34. The ultrasonic energy output unit 34 is connected to a later-described ultrasonic transducer 43 of the energy treatment instrument 12.

In addition, an unshown foot switch or hand switch is connected to the ultrasonic energy source 16. Thus, the foot switches or hand switches are connected to the high-frequency energy source 14 and the ultrasonic energy source 16, respectively. Alternatively, it is also preferable that a common foot switch or hand switch be connected to the high-frequency energy source 14 and the ultrasonic energy source 16.

As shown in FIG. 1A, the energy treatment instrument 12 includes a handle 42, a shaft 44 and a treatment portion 46.

The handle 42 is substantially L-shaped. The proximal end of the shaft 44 is disposed at one end of the handle 42. On the other hand, the other end of the handle 42 serves as a grip portion gripped by an operator (user of the energy treatment instrument 12). The handle 42 is provided with a first knob (lengthwise feed lever) 42a side by side with the other end (grip portion) of the handle 42, and the first knob 42a serves to operate the later-described first treating portion 62 of the treatment portion 46. If the first knob 42a is brought closer to or away from the other end of the handle 42, a later-described sheath 54 axially moves. The handle 42 is provided, at its one end, with a second knob (lengthwise feed lever) 42b for moving a later-described second treating portion 64 along the axial direction of the shaft 44. The second knob 42b can be brought closer to or away from the operator.

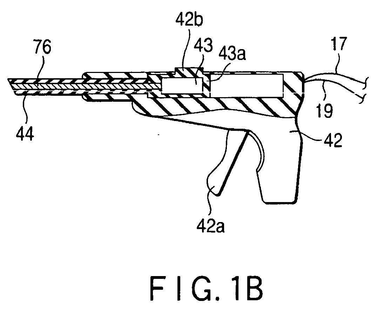

As shown in FIG. 1B, the ultrasonic transducer 43 is provided within the handle 42. A housing 43a of the ultrasonic transducer 43 is formed integrally with the second knob 42b of the handle 42. Further, the proximal end of a later-described ultrasonic probe 76 of the second treating portion 64 of the treatment portion 46 is connected to the ultrasonic transducer 43. Then, when energy is supplied from the ultrasonic energy source 16 to the ultrasonic transducer 43 through a later-described ultrasonic energy conducting line 20a, the ultrasonic transducer 43 ultrasonically vibrates. That is, electric energy is converted to mechanical energy. Then, the vibrations of the ultrasonic transducer 43 are transmitted from the proximal end to distal end of the ultrasonic probe 76.

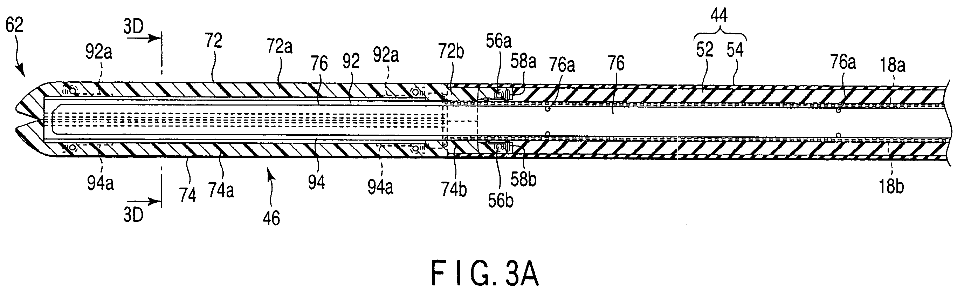

As shown in FIG. 3A to FIG. 3D, the shaft 44 includes a cylindrical member 52, and the sheath 54 slidably provided outside the cylindrical member 52. The cylindrical member 52 is fixed at its proximal end to one end of the handle 42. The sheath 54 is slidable along the axial direction of the cylindrical member 52 by the operation of the first knob 42a at the other end of the handle 42.

As shown in FIG. 1A, the treatment portion 46 includes the first treating portion 62 and the second treating portion 64. The first treating portion 62 has the first and second holding members (a pair of holding members) 72, 74 which can open and close with respect to each other. The second treating portion 64 has the ultrasonic probe 76 which can be provided between the first and second holding members 72, 74.

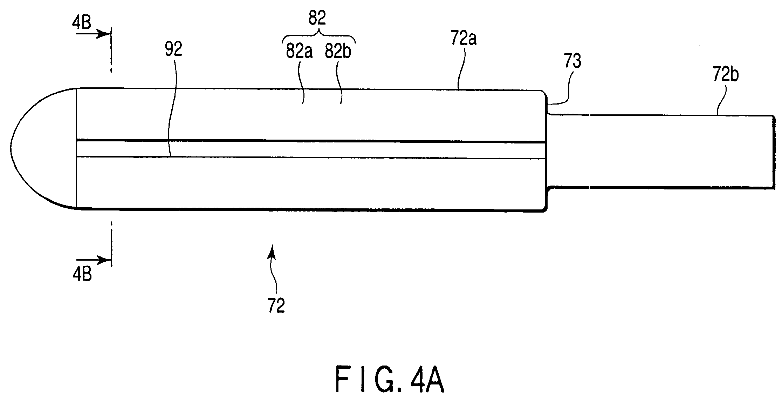

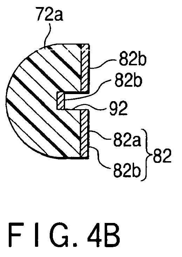

In addition, the second holding member 74 has the same structure as the first holding member 72 shown in FIG. 4A and FIG. 4B, and therefore, the structure of the first holding member 72 is mainly described for representation. Moreover, the detailed structure of the second holding member 74 is not shown, but is properly provided with numerals for the purpose of explanation.

As shown in FIG. 1A, the first and second holding members 72, 74 are provided at the distal end of the shaft 44. The first holding member 72 integrally has a main body 72a and a base 72b. The second holding member 74 integrally has a main body 74a and a base 74b. In addition, the distal ends of the main bodies 72a, 74a of the first and second holding members 72, 74 are most distal to the handle 42, and the proximal ends of the main bodies 72a, 74a are most proximal to the handle 42. The first and second holding members 72, 74 have longitudinal axes determined by the distal ends and the proximal ends. Later-described grooves 92, 94 are formed along the longitudinal axes.

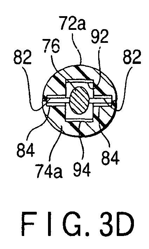

As shown in FIG. 3D and FIG. 4B, the outer surfaces of the main bodies 72a, 74a of the first and second holding members 72, 74 are smoothly curved. Although not shown, the outer surfaces of the bases 72b, 74b of the first and second holding members 72, 74 are also smoothly curved. When the second holding member 74 is closed with respect to the first holding member 72, the cross sections of the main bodies 72a, 74a of the holding members 72, 74 are substantially circular or substantially elliptic as a whole. When the second holding member 74 is closed with respect to the first holding member 72, the cross sections of the bases 72b, 74b are substantially cylindrical as a whole. In this state, the outside diameters of the proximal ends of the main bodies 72a, 74a of the first and second holding members 72, 74 are greater than the outside diameters of the first and second bases 72b, 74b. Thus, steps 73 are formed between the first and second main bodies 72a, 74a and the bases 72b, 74b. The distal end of the sheath 54 of the shaft 44 comes into or out of contact with the steps 73 by the operation of the first knob 42a.

Here, when the second holding member 74 is closed with respect to the first holding member 72, the outer peripheral surfaces, which are substantially circular or substantially elliptic as a whole, of the bases 72b, 74b of the first and second holding members 72, 74 are substantially flush with or slightly greater in diameter than the outer peripheral surface of the distal end of the cylindrical member 52. Therefore, the sheath 54 of the shaft 44 can be slid over the cylindrical member 52 so that the bases 72b, 74b of the first and second holding members 72, 74 are covered with the distal end of the sheath 54.

The proximal ends of the bases 72b, 74b of the first and second holding members 72, 74 are both supported rotatably around the distal end of the cylindrical member 52 of the shaft 44 in a direction perpendicular to the axial direction of the shaft 44 by support pins 56a, 56b which are disposed at the distal end of the cylindrical member 52. These support pins 56a, 56b are provided at the distal end of the cylindrical member 52 in parallel with each other. The bases 72b, 74b of the first and second holding members 72, 74 are rotated around the axes of the support pins 56a, 56b so that the main bodies 72a, 74a of the holding members 72, 74 can be opened or closed with respect to each other. The bases 72b, 74b of the first and second holding members 72, 74 are respectively urged by elastic members 58a, 58b such as leaf springs so that later-described holding surfaces 82, 84 of the main bodies 72a, 74a to be in contact with the living tissue are opened with respect to the position where the holding surfaces are in contact with each other. Actually, as shown in FIG. 3A to FIG. 3C, the elastic members 58a, 58b are provided on the outer peripheries of the support pins 56a, 56b provided at the distal end of the cylindrical member 52. Therefore, the bases 72b, 74b of the first and second holding members 72, 74 are urged in a direction to open.

Thus, when the first knob 42a is operated to move the distal end of the sheath 54 (forward) distally with respect to the operator, force is applied so that the bases 72b, 74b may be closed by the distal end of the sheath 54. Then, the first and second holding members 72, 74 close against the urging force by the elastic members 58a, 58b. In this case, if the living tissue is not in contact with the holding surfaces 82, 84 of the main bodies 72a, 74a of the first and second holding members 72, 74, the holding surfaces 82, 84 are in contact with each other. On the other hand, when the first knob 42a is operated to move the distal end of the sheath 54 (backward) proximally with respect to the operator, there is no force of the distal end of the sheath 54 to close the bases 72b, 74b, that is, the first holding member 72 and the second holding member 74 are opened due to the urging force by the elastic members 58a, 58b.

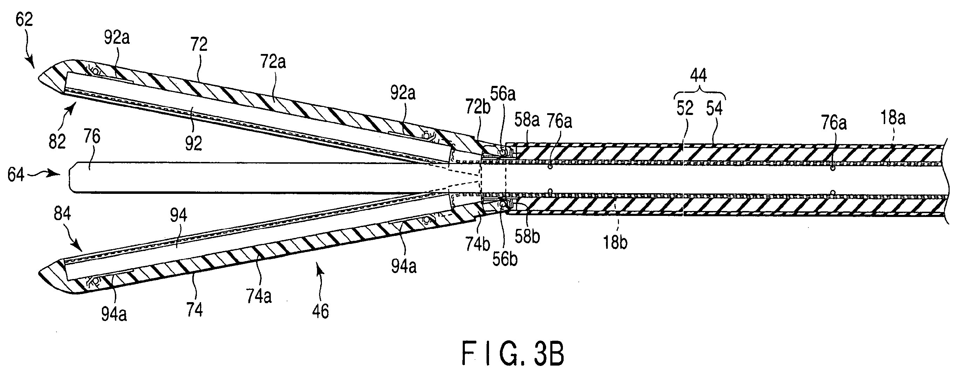

As shown in FIG. 3B, the first holding surface 82 for holding a treatment target living tissue is formed on the side of the main body 72a of the first holding member 72 proximate to the main body 74a of the second holding member 74. The second holding surface 84 for holding the treatment target living tissue is formed on the side of the main body 74a of the second holding member 74 proximate to the main body 72a of the first holding member 72. The first holding surface 82 has a first contact surface 82a which comes into contact with the living tissue when holding the living tissue, and a first electrode 82b as an energy emitter for emitting energy to the living tissue. The second holding surface 84 has a second contact surface 84a which comes into contact with the living tissue when holding the living tissue, and a second electrode 84b as an energy emitter for emitting energy to the living tissue.

As shown in FIG. 4A, the first and second contact surfaces 82a, 84a are flat. The first contact surface 82a is provided with the flat-plate-shaped first electrode 82b, as shown in FIG. 4B. The second contact surface 84a is provided with the flat-plate-shaped second electrode 84b. The first and second electrodes 82b, 84b are provided over the substantially entire surfaces of the first and second contact surfaces 82a, 84a except for their distal ends, as shown in FIG. 4A. In addition, the end face (side surface) of the first electrode 82b is aligned with the side surface of the main body 72a of the first holding member 72. The end face (side surface) of the second electrode 84b is aligned with the side surface of the main body 74a of the second holding member 74.

The first groove (recess) 92 where the ultrasonic probe 76 is disposed is formed in the center of the contact surface 82a (first electrode 82b) of the holding surface 82 of the main body 72a of the first holding member 72. Similarly to the main body 72a, the second groove 94 is formed in the center of the contact surface 84a (second electrode 84b) of the holding surface 84 of the main body 74a of the second holding member 74 at a position opposite to the first groove 92 of the first holding member 72. The width of the grooves 92, 94 of the first and second main bodies 72a, 74a is greater than the width of the ultrasonic probe 76. Moreover, the depth of the grooves 92, 94 of the first and second main bodies 72a, 74a is greater than half of the height of the ultrasonic probe 76. Thus, when the first treating portion 62 is closed, that is, when the first and second holding members 72, 74 are closed, the ultrasonic probe 76 is stored movably in and out without contacting the grooves 92, 94.

In addition, as shown in FIG. 4B, in order to apply sufficient high-frequency energy to the surface where the living tissue is removed, the groove 92 of the first holding member 72 is also provided with the first electrode (high-frequency electrode) 82b, and the groove 94 of the second main body 74a is also provided with the second electrode (high-frequency electrode) 84b. The first electrode 82b of the groove 92 of the first holding member 72 is formed to be discontinuous with but to have the same potential as the electrode 82b of the first contact surface 82a of the first holding member 72. Similarly, the second electrode 84b of the groove 94 of the second holding member 74 is formed to be discontinuous with but to have the same potential as the electrode 84b of the second contact surface 84a of the second holding member 74.

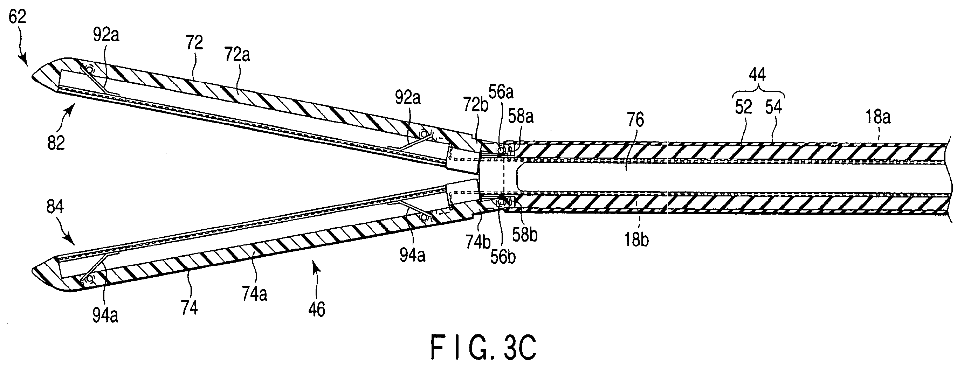

Elastic members 92a, 94a such as leaf springs (see FIG. 3A to FIG. 3C) are provided on the rear surfaces of the electrodes 82b, 84b disposed in the grooves 92, 94. The elastic members 92a, 94a can act together with the operation of the second knob 42b. When the ultrasonic probe 76 is between the main bodies 72a, 74a of the first and second holding members 72, 74 as shown in FIG. 3A and FIG. 3B, the electrodes 82b, 84b disposed in the grooves 92, 94 are drawn in the main bodies 72a, 74a. When the ultrasonic probe 76 is removed from between the main bodies 72a, 74a of the first and second holding members 72, 74 and thus drawn in the shaft 44 as shown in FIG. 3C, the electrodes 82b, 84b disposed in the grooves 92, 94 are pressed by the elastic members 92a, 94a and are flush with the holding surfaces 82, 84.

Thus, when the second knob 42b is disposed distally with respect to the operator, the elastic members 92a, 94a act together with the second knob 42b in such a manner as to draw the electrodes 82b, 84b disposed in the grooves 92, 94 into the main bodies 72a, 74a. On the other hand, when the second knob 42b is disposed proximally with respect to the operator, the elastic members 92a, 94a act together with the second knob 42b so that the electrodes 82b, 84b disposed in the grooves 92, 94 may be flush with the holding surfaces 82, 84 of the main bodies 72a, 74a. That is, the electrodes 82b, 84b disposed in the grooves 92, 94 are on the same surface as the electrodes 82b, 84b disposed on the holding surfaces 82, 84. Therefore, the elastic members 92a, 94a bring the electrodes 82b, 84b disposed in the grooves 92, 94 out of an urged state together with the forward movement of the ultrasonic probe 76, and bring the electrodes 82b, 84b disposed in the grooves 92, 94 into an urged state together with the backward movement of the ultrasonic probe 76. At the same time, the living tissue is also pressed by the electrodes 82b, 84b disposed in the grooves 92, 94.

As shown in FIG. 3D, the cross section of the ultrasonic probe 76 is, for example, circular, but is permitted to have various shapes such as a polygonal shape. The sectional shape of the grooves 92, 94 of the holding surfaces 82, 84 formed by the first and second holding members 72, 74 is preferred to be similar to that of the ultrasonic probe 76, but is permitted to be various shapes such as circular, elliptic and polygonal shapes.

First and second conducting lines 18a, 18b are provided inside the cylindrical member 52 of the shaft 44, inside the handle 42, and within the cable 17. The first and second conducting lines 18a, 18b are connected on one end to the first and second electrodes 82b, 84b and connected on the other end to the connector 17a of the cable 17. Thus, energy can be supplied to the first electrode 82b and the second electrode 84b from the high-frequency energy source 14 through the connector 17a and the first and second conducting lines 18a, 18b.

At the same time, the first and second high-frequency electrodes 82b, 84b serve as sensors, and thus measure a current, voltage, etc. flowing the first and second high-frequency electrodes 82b, 84b through the living tissue, and then input relevant signals to the detecting portion 22 of the high-frequency energy source 14 through the first and second conducting lines 18a, 18b.

The ultrasonic energy conducting line 20a is provided inside the handle 42 and within the cable 19. The ultrasonic energy conducting line 20a is connected on one end to the ultrasonic transducer 43 and connected on the other end to the connector 19a of the cable 19. Thus, energy can be supplied to the ultrasonic transducer 43 from the ultrasonic energy source 16 through the connector 19a and the ultrasonic energy conducting line 20a.

The ultrasonic probe 76 described above is provided inside the cylindrical member 52 of the shaft 44. Insulating supports 76a such as O-rings formed of, for example, rubber are provided on the outer peripheral surface of the ultrasonic probe 76 at the positions of vibration nodes in the transmission of ultrasonic vibrations from the ultrasonic transducer 43. This makes it possible to prevent a direct contact between the outer peripheral surface of the ultrasonic probe 76 and the inner peripheral surface of the cylindrical member 52.

Furthermore, the housing 43a of the ultrasonic transducer 43 at the proximal end of the ultrasonic probe 76 is fixed to the second knob 42b of the handle 42. Thus, when the second knob 42b is moved distally with respect to the operator, the ultrasonic probe 76 for removing surface tissues of the target living tissues by cavitation effects projects from the distal end of the cylindrical member 52 and is then located between the main bodies 72a, 74a of the first and second holding members 72, 74, as shown in FIG. 3A and FIG. 3B. When the second knob 42b is moved toward the operator, the ultrasonic probe 76 located between the first and second holding members 72, 74 axially moves toward the operator, as shown in FIG. 3C. As a result, the distal end of the ultrasonic probe 76 is stored in the distal end (treatment side end) of the cylindrical member 52.

In addition, the length of the ultrasonic probe 76 located between the first and second holding members 72, 74 is formed so that the ultrasonic probe 76 may not extend beyond the distal ends of the first and second high-frequency electrodes 82b, 84b provided in the main bodies 72a, 74a of the first and second holding members 72, 74. That is, the distal end of the ultrasonic probe 76 is prevented from contacting the distal ends of the grooves 92, 94. Moreover, the width of the ultrasonic probe 76 disposed between the first and second holding members 72, 74 is smaller than the width of the first and second high-frequency electrodes 82b, 84b. Thus, the tissue can be removed in a more limited manner in the treatment with the ultrasonic probe 76 than in the treatment with the first and second high-frequency electrodes 82b, 84b.

Now, the effects of the medical treatment apparatus 10 according to this embodiment are described.

When unshown power switches provided in the high-frequency energy source 14 and the ultrasonic energy source 16 are, for example, pressed and turned on, the high-frequency energy source 14 and the ultrasonic energy source 16 become operable (on standby).

As shown in FIG. 3A, the second holding member 74 is closed with respect to the first holding member 72, in which state the treatment portion 46 and the shaft 44 of the energy treatment instrument 12 are inserted into, for example, an abdominal cavity through an abdominal wall. The treatment portion 46 of the energy treatment instrument 12 is put face-to-face with the target living tissues (treatment target). At this point, the distal end of the ultrasonic probe 76 may be inside or outside the cylindrical member 52 of the shaft 44.

The first knob 42a of the handle 42 is operated so that the target living tissues may be held (grasped) by the first holding member 72 and the second holding member 74. At the same time, the sheath 54 is moved with respect to the cylindrical member 52 toward the operator side of the shaft 44. Owing to the urging force by the elastic members 58a, 58b, a cylindrical space between the first and second bases 72b, 74b can not be maintained, and the first holding member 72 and the second holding member 74 open with respect to each other. Here, the first holding member 72 and the second holding member 74 simultaneously open at the same angle to the axial direction (central axis) of the shaft 44.

Then, the second knob 42b is operated to extend the ultrasonic probe 76 with respect to the distal end of the cylindrical member 52 of the shaft 44. At the same time, one of the two treatment target living tissues (one living tissue) is disposed between the first high-frequency electrode 82b of the first holding member 72 and the ultrasonic probe 76, and the other living tissue to be joined to the former living tissue is disposed between the second high-frequency electrode 84b of the second holding member 74 and the ultrasonic probe 76. That is, the ultrasonic probe 76 is disposed between the target living tissues so that the ultrasonic probe 76 is held by the two living tissues, and the living tissues are disposed between the first and second holding members 72, 74.

In this state, the first knob 42a of the handle 42 is operated. At the same time, the sheath 54 is moved with respect to the cylindrical member 52 toward the distal side of the shaft 44. The space between the first and second bases 72b, 74b is closed and formed into a cylindrical shape by the sheath 54 against the urging force by the elastic members 58a, 58b. As a result, the main body 72a formed integrally with the base 72b of the first holding member 72 is closed with respect to the main body 74a formed integrally with the base 74b of the second holding member 74. Thus, the target two living tissues are held (grasped) between the first holding member 72 and the second holding member 74.

When tubular-shaped organs such as blood vessels or intestinal tracts are joined together, it is necessary to insert the ultrasonic probe 76 into the tube such as blood vessels or intestinal tracts. It is also possible to insert the ultrasonic probe 76 into the tube while ultrasonically vibrating the ultrasonic probe 76. The ultrasonic probe 76 also has a function of physical puncture with no application of energy. Therefore, the ultrasonic probe 76 can be disposed on the joint surfaces of the tube after the first and second holding members 72, 74 are closed.

In this case, the target living tissues are in contact with both the first electrode 82b of the first holding member 72 and the second electrode 84b of the second holding member 74. Tissues around the target living tissues are in close contact with both the holding surface (contact surface, grasping surface) 82 of the first holding member 72 and the holding surface (contact surface, grasping surface) 84 of the second holding member 74 as well.

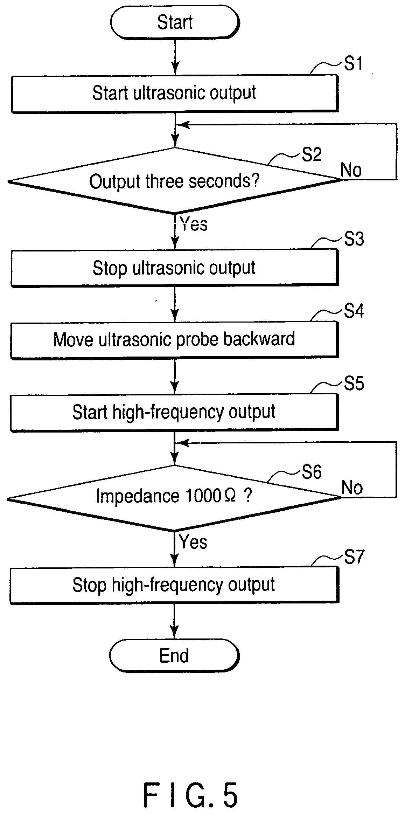

In this state, a foot switch or hand switch connected to the high-frequency energy source 14 and a foot switch or hand switch connected to the ultrasonic energy source 16 are properly operated. The effects of the medical treatment apparatus 10 are described below in detail along with a flowchart shown in FIG. 5.

Energy is supplied to the ultrasonic transducer 43 from the ultrasonic energy source 16 via the ultrasonic energy conducting line 20a in the cable 19. The electric energy output from the ultrasonic energy source 16 is converted into ultrasonic vibrations by the ultrasonic transducer 43. Thus, the ultrasonic vibrations are transmitted to the proximal end of the ultrasonic probe 76 (S1). Then, the living tissues are cavitated by the distal end of the ultrasonic probe 76 using the ultrasonic vibrations transmitted from the proximal end to distal end of the ultrasonic probe 76.

Cells in the surfaces of the living tissues are broken by the cavitation so that epithelial cells in the surface portion are desquamated (removed). The desquamated living tissues are forced out of the first and second holding members 72, 74 due to the holding pressure of the first and second holding members 72, 74. Collagen (although not described in particular, it will hereinafter be assumed that collagen contains collagen fibers) is a living body component that is difficult to break due to cavitation caused by an ultrasonic energy treatment, and therefore remains even after the ultrasonic treatment. As a result, collagen is exposed in the joint surfaces of the living tissues.

In this case, if the side surface of the distal end of the ultrasonic probe 76 includes uneven or curved shapes, cavitation is also caused on the side surface of the ultrasonic probe 76, so that the living tissue in contact with the side surface of the ultrasonic probe 76 can be treated. When the side surface of the ultrasonic probe 76 has no unevenness and curved shapes, cavitation is caused in front of the distal end of the ultrasonic probe 76. In this case, the second knob 42b of the handle 42 is operated to bring the ultrasonic probe 76 closer to or away from the operator during the output of ultrasonic waves. Consequently, more collagen can be exposed in the joint surfaces of the living tissues over as entire length of the living tissues held by the holding members 72, 74 as possible. In addition, the ultrasonic probe 76 may be automatically moved forward and backward (brought closer to and away from the operator) together with the output from the ultrasonic energy output unit 34.

The treatment by the ultrasonic vibrations is limited by time, for example, three seconds (S2). Therefore, the ultrasonic output controller 32 automatically stops the output from the ultrasonic energy output unit 34 of the ultrasonic energy source 16 after a given period of time from the start of the output (S3).

After the output from the ultrasonic energy output unit 34 has been stopped, the second knob 42b of the handle 42 is moved to the side proximate to the operator. That is, the ultrasonic probe 76 located between the holding members 72, 74 is moved backward to the side proximate to the operator. Then, the distal end of the ultrasonic probe 76 is stored in the distal end of the cylindrical member 52 (S4).

At the same time, as shown in FIG. 3C, the electrodes 82b, 84b disposed in the grooves 92, 94 are brought into an urged state by the elastic members 92a, 94a together with the backward movement of the ultrasonic probe 76. Thus, the living tissues are pressed by the electrodes 82b, 84b disposed in the grooves 92, 94 of the first and second holding members 72, 74. As a result, there is no longer the space where the ultrasonic probe 76 is disposed between the joint surfaces of the living tissues, so that exposed collagens can be brought into contact with each other by the ultrasonic probe 76.

Then, the foot switch or hand switch connected to the high-frequency energy source 14 is operated. Energy is supplied to the first high-frequency electrode 82b and the second high-frequency electrode 84b from the high-frequency energy source 14 through the first and second conducting lines 18a, 18b within the cable 17.

A high-frequency current is applied across the first high-frequency electrode 82b provided in the first holding member 72 and the second high-frequency electrode 84b provided in the second holding member 74 via the target living tissues. That is, high-frequency energy is supplied to the living tissues in contact with the electrodes 82b, 84b out of the living tissues held between the first and second holding members 72, 74 (S5). Thus, the high-frequency energy is supplied to the target living tissues grasped between the electrodes 82b, 84b. As a result, the target living tissues in contact with the electrodes 82b, 84b generate heat. That is, Joule heat is generated within the living tissues grasped between the electrodes 82b, 84b so that the living tissues themselves are heated. The high-frequency energy denatures proteins contained in the living tissues including collagens in the tissue surfaces exposed by the ultrasonic energy. At the same time, the living tissues themselves generate heat and are dehydrated. Consequently, proteins bond with each other, such that components constituting the living tissues bond with each other at the junction of the living tissues. That is, the target living tissues are gradually denatured and dehydrated and thus united.

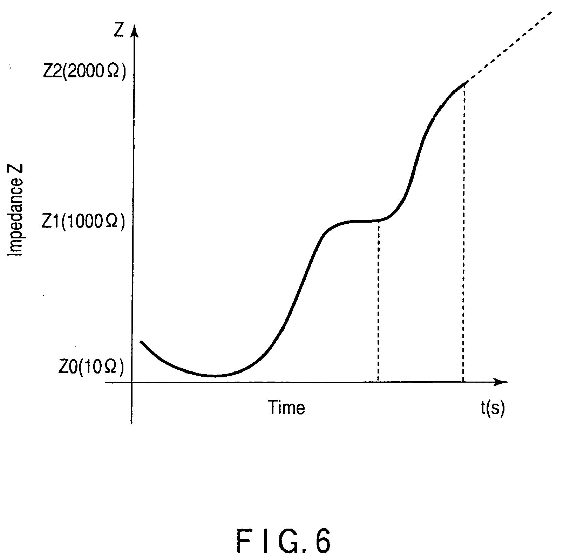

Simultaneously with the start of the treatment of the living tissues by the high-frequency energy, the detecting portion 22 of the high-frequency energy source 14 detects the impedance Z of the living tissues in contact with the high-frequency electrodes 82b, 84b of the first and second holding members 72, 74. The impedance Z at the beginning of the treatment shown in FIG. 6 (initial value) is, for example, about 50[Ω], which however varies depending on the size and shape of the electrodes 82b, 84b. Then, as high-frequency energy is applied to the living tissues and the living tissues are denatured and dehydrated, the value of the impedance Z once drops from about 50[Ω], and then rises, as shown in FIG. 6. Such a rise in the value of the impedance Z represents that the living tissues are losing water and drying.

Then, it is judged whether the calculated impedance Z has exceeded, for example, 1000[Ω] (not limited to this value and any value can be set) set as the threshold value in the high-frequency output controller 24 (S6). When the impedance Z is judged to have exceeded a threshold value of 1000[Ω], the high-frequency output controller 24 stops the output of the high-frequency electric power from the high-frequency output unit 26 (S7).

That is, joining of the living tissues using the ultrasonic energy and the high-frequency energy is ended.

The series of steps of such a control method shown in the flowchart of FIG. 5 is performed when the foot switches or hand switches connected to the ultrasonic energy source 16 and the high-frequency energy source 14 are kept pressed. On the other hand, when the foot switches or hand switches are released, the treatment of the living tissues is forcibly ended. It goes without saying that the treatment is automatically ended when the impedance Z has exceeded a threshold value of 1000[Ω]. In this case, it is preferable that the user be informed of the end of the treatment such as the stopped generation of the ultrasonic vibrations or the stopped supply of the high-frequency energy by a buzzer, light or some other indication. It is also preferable to change, for example, the tone of the buzzer between the ultrasonic treatment and the high-frequency treatment.

Although the generation of the ultrasonic vibrations by the ultrasonic probe 76, the backward movement of the ultrasonic probe 76 and the output to the living tissues between the first and second holding members 72, 74 are manually performed here, the series of operations may be automatically performed. In this case, although not shown, the high-frequency output controller 24 of the high-frequency energy source 14 is preferably connected to the ultrasonic output controller 32 of the ultrasonic energy source 16 by a cable. Such connection enables improved transfer of electric signals between the high-frequency energy source 14 and the ultrasonic energy source 16. Thus, the series of operations including the generation of the ultrasonic vibrations by the ultrasonic probe 76, the backward movement of the ultrasonic probe 76 and the output to the living tissues between the first and second holding members 72, 74 can be performed in a shorter time than when manually performed. Moreover, the series of operations can be preferably ended by pressing a common foot switch or hand switch of the high-frequency energy source 14 and the ultrasonic energy source 16. It goes without saying that the treatment is forcibly ended when the common foot switch or hand switch is released in the middle of the treatment.

It is also advantageous to provide an idle period in the high-frequency output or to repeat lower outputs and high outputs. That is, the treatment may be performed with the threshold value of the impedance Z set at, for example, 500[Ω]. Then, after an idle period of several seconds to wait for the drop of the impedance Z, the treatment (the application of electricity to the living tissues) may be repeatedly performed in such a manner as to sequentially increase the threshold value by 100[Ω] up to 1000[Ω].

Furthermore, as to a termination condition for a treatment, the treatment may be automatically ended not only after judging whether the threshold value of the impedance Z has exceeded the threshold value set as the termination condition but also after output of the high-frequency energy for a certain period of time.

As described above, the following can be said according to this embodiment.

In the present embodiment, first, cells in the surfaces of the target living tissues can be fractured by the treatment with ultrasonic output. In this case, if the surface of the ultrasonic probe 76 includes uneven or curved shapes, cavitation can also be caused on the side surface of the ultrasonic probe 76, so that the living tissue on the side surface of the ultrasonic probe 76 can be treated. When the side surface of the ultrasonic probe 76 includes no unevenness or curved shapes, cavitation is only caused in front of the ultrasonic probe 76. In this case, the second knob 42b of the handle 42 is operated to move the ultrasonic probe 76 forward or backward during the output of ultrasonic waves, such that the living tissues can be broken over the entire length of the target living tissues. The ultrasonic probe 76 can be moved forward or backward manually or automatically.

Collagen is a component that is more difficult to fracture due to cavitation than other living tissue components such as cells, and therefore remains even after the ultrasonic treatment. The fractured living tissues are then excluded by pressure to the side of the holding members 72, 74 in the step of holding the living tissues. As a result, collagens can be exposed in the surfaces (joint surfaces) of the target living tissues.

Then, the ultrasonic probe 76 is brought out of contact with the joint surfaces (treatment surfaces) of the living tissues and stored in the sheath 54, such that the exposed collagens can be brought into close contact with each other. Such close contact between collagens enables denatured collagen molecules to be bonded together during the subsequent treatment by the high-frequency output.

In the subsequent treatment by the high-frequency output, the collagens exposed and in close contact with each other are denatured by Joule heat and bonded together, as described above. At the same time, heat generation is caused to the living tissues to evaporate the water contained in the living tissues.

Collagen is a protein that has the strongest bonding force among the proteins present in a living body. Exposing the collagen in the joint surfaces enables firmer bonding of the living tissues than the bonding of living tissues including cells present in the joint surfaces. Moreover, the difference in the kind of proteins present in the surface of the tissue is one reason for the variation in the bonding force of different tissues in a living body. If collagens can always be exposed in every living tissue, the composition of the living tissues in the joint surfaces can be uniform, and stable tissue bonding is therefore enabled. That is, it is possible to reduce the variation of tissue bonding strength due to the difference of species of cells present in the surface or the difference of structure of tissues depending on the kind of organs.

Collagens are brought close to each other and joined together, so that fibroblasts easily migrate from neighboring tissues. This enables early healing of tissues and creation of an environment that improves the strength of living tissues early after surgery.

The present embodiment makes it possible to provide the energy treatment instrument 12 having such effects and assuring high safety.

In addition, although the living tissues are joined together by the energy treatment instrument 12 in the example described here, the living tissues can also be simply coagulated.

Furthermore, when moving the ultrasonic probe 76 forward and backward, it is also preferable to rotate the ultrasonic probe 76 around its axis. For example, a motor (included in the numeral 43 in FIG. 1B) is provided in the housing 43a of the ultrasonic transducer 43, such that the ultrasonic probe 76 can be rotated for each ultrasonic transducer 43. When the ultrasonic probe 76 is thus rotatable, it is possible to more easily remove epithelial cells and expose, for example, collagen.

Moreover, in this embodiment, the foot switch or hand switch is provided in each of the high-frequency energy source 14 and the ultrasonic energy source 16, and the ultrasonic treatment and the high-frequency treatment are performed in this order. However, when the foot switch or hand switch of the high-frequency energy source 14 is to be operated before the foot switch or hand switch of the ultrasonic energy source 16, it is also preferable to set so that no energy may be output from the high-frequency output unit 26. In this case, it is naturally possible to output energy from the high-frequency output unit 26 after the output of energy from the ultrasonic output unit 34.

The medical treatment apparatus 10 has been described above with FIG. 1A to FIG. 6 in the present embodiment, but the present invention is not limited to this. Each component can be replaced with any component having a similar function. Although not shown, similar effects can be obtained if the high-frequency electrodes 82b, 84b of the holding members 72, 74 are replaced with, for example, heater elements (heaters). In this case, the heater elements can serve as sensors as described above. Moreover, the heater element may be combined with the high-frequency electrode.

Although the impedance Z of the target living tissues is detected to recognize the state of the target living tissues in the present embodiment, the biological information is not limited to the impedance Z. For example, other electric information such as an electric power value or a phase is also permitted. That is, the biological information includes, for example, a current, a voltage and electric power for calculating the impedance Z, the impedance Z calculated therefrom, and phase information.

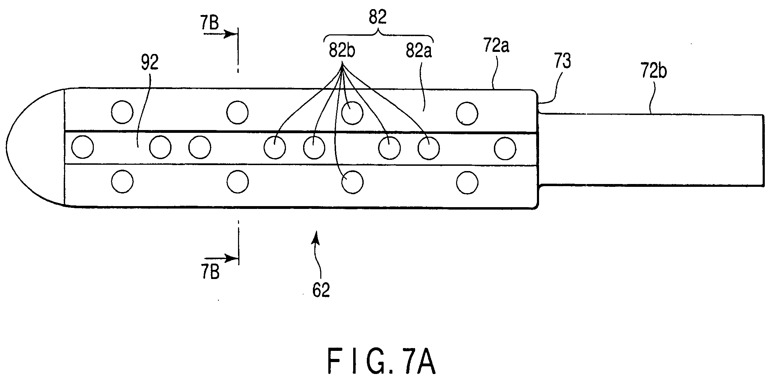

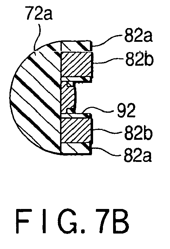

It is also preferable that the electrodes 82b, 84b of the holding members 72, 74 be formed and arranged as shown in FIG. 7A and FIG. 7B. In this case, the electrodes 82b, 84b are circular. Moreover, some of the electrodes 82b disposed in the groove 92 may be arranged proximately to each other in the axial direction instead of being arranged at equal intervals. When the electrodes 82b of the first holding member 72 are thus arranged, it is possible to perform a treatment wherein current density is increased for the opposite electrode 84b of the second holding member 74.

Moreover, in the case described in this embodiment, the second knob 42b separate from the first knob 42a is used to move the ultrasonic probe 76 with respect to the shaft 44, as shown in FIG. 1A and

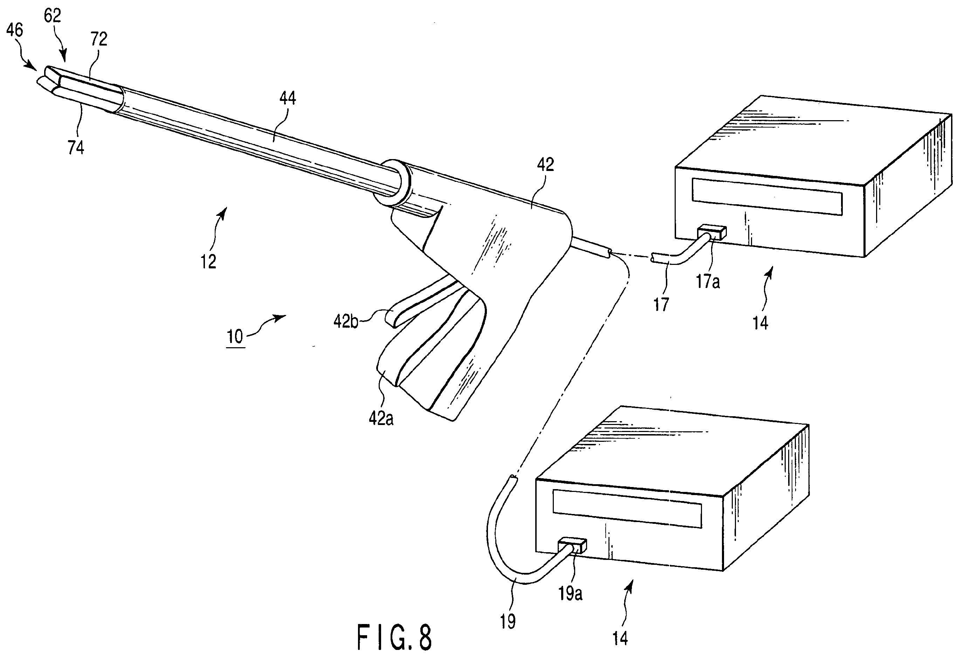

FIG. 1B. Otherwise, it is also preferable that the first knob 42a and the second knob 42b be provided side by side. When the second knob 42b shown in FIG. 8 is brought closer to the other end of the handle 42, the distal end of the ultrasonic probe 76 is drawn into the distal end of the shaft 44. When the second knob 42b is brought away from the other end of the handle 42, the distal end of the ultrasonic probe 76 projects from the distal end of the shaft 44 and is located between the first and second holding members 72, 74.

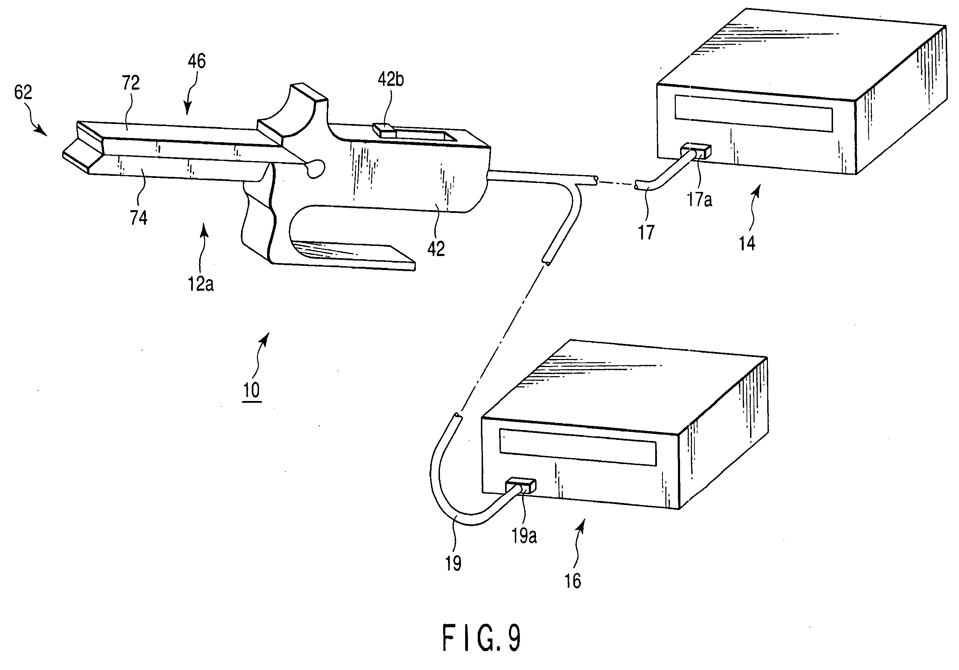

Furthermore, the linear energy treatment instrument 12 (see FIG. 1A) for treating living tissues in an abdominal cavity (in the body) through an abdominal wall has been described as an example in this embodiment. However, it is also possible to use, for example, an open linear energy treatment instrument (medical treatment instrument) 12a shown in FIG. 9 for taking treatment target living tissues out of the body through an abdominal wall and then treating the same. This energy treatment instrument 12a includes a handle 42 and a treatment portion 46. That is, the energy treatment instrument 12a has no shaft 44 (see FIG. 1A) in contrast with the energy treatment instrument 12 for treating through an abdominal wall. On the other hand, a member having the same function as the shaft 44 is provided in the handle 42. Thus, the energy treatment instrument 12a can be used similarly to the above-described energy treatment instrument 12 shown in FIG. 1A.

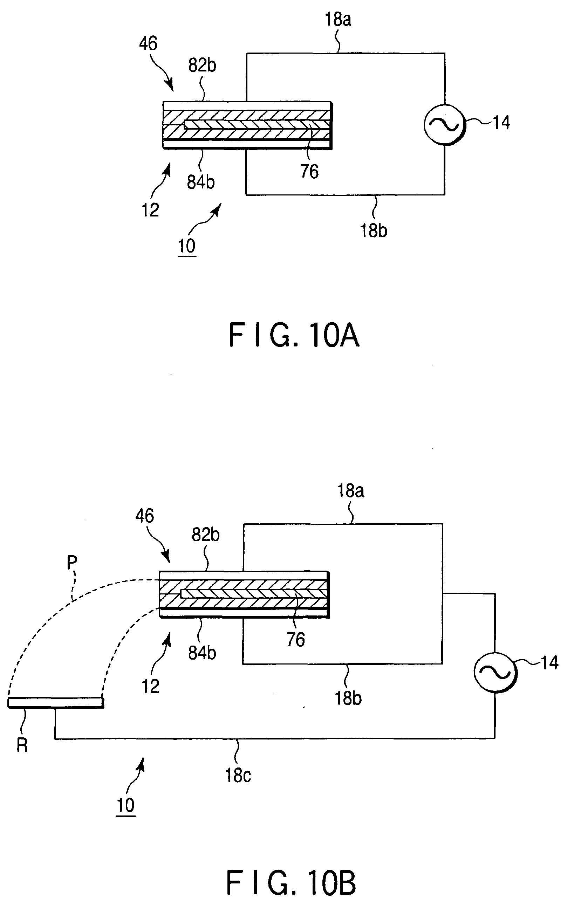

In addition, a bipolar treatment has been described as schematically shown in FIG. 10A in connection with the high-frequency treatment in the first embodiment. That is, electricity is applied to the living tissues between the first and second electrodes 82b, 84b in the case described. Here, it is also preferable to perform a monopolar treatment as shown in FIG. 10B. In this case, a return electrode plate R is attached to a patient P to be treated. The return electrode plate R is connected to the high-frequency energy source 14 via a conducting line 18c.

Then, as shown in FIG. 10B, when the first and second electrodes 82b, 84b are homopolar, electricity is applied to the return electrode plate R and the living tissue between the first and second electrodes 82b, 84b. In this case, the area of the living tissue in contact with the first and second electrodes 82b, 84b is sufficiently smaller than the area of the living tissue in contact with the return electrode plate R. Therefore, energy density is higher for the living tissue in contact with the first and second electrodes 82b, 84b. Thus, the living tissue held between the first and second electrodes 82b, 84b is treated.

### [Second Embodiment]

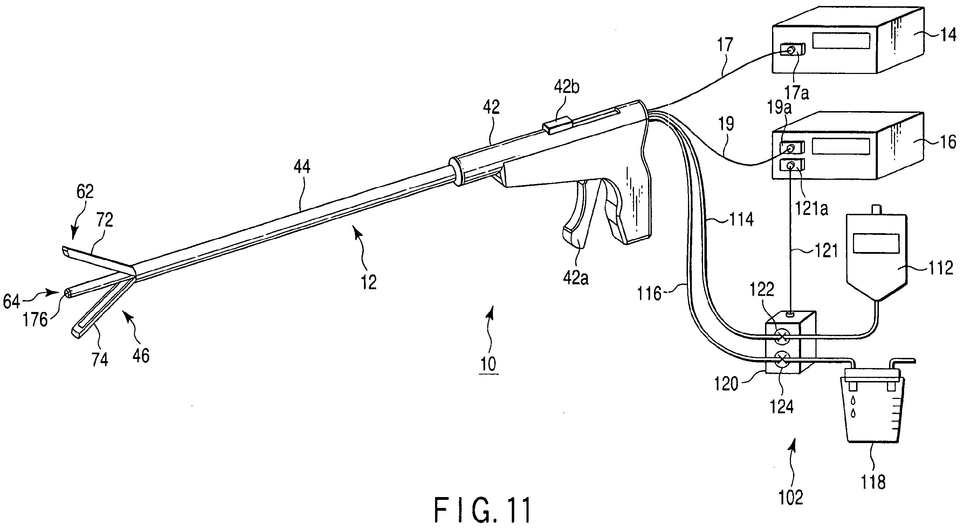

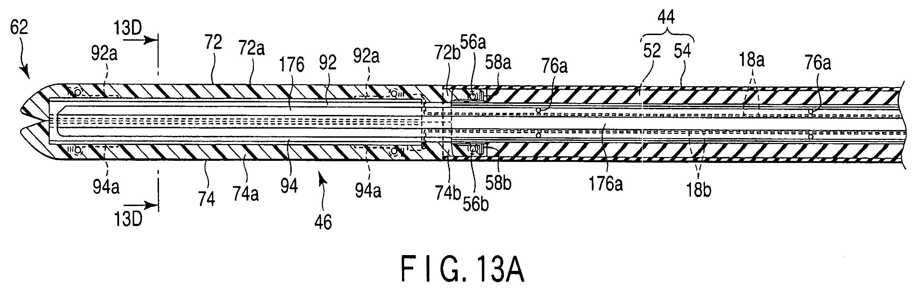

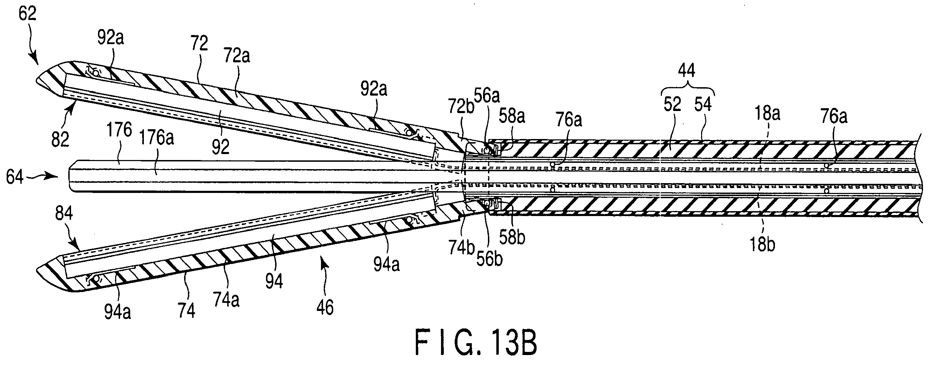

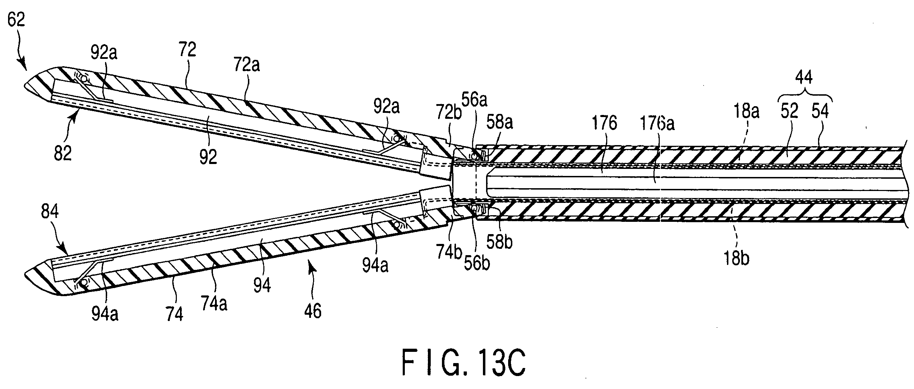

Next, a second embodiment is described with FIG. 11 to FIG. 15. This embodiment is a modification of the first embodiment, and the same parts as the parts described in the first embodiment are provided with the same numerals and are not described in detail.

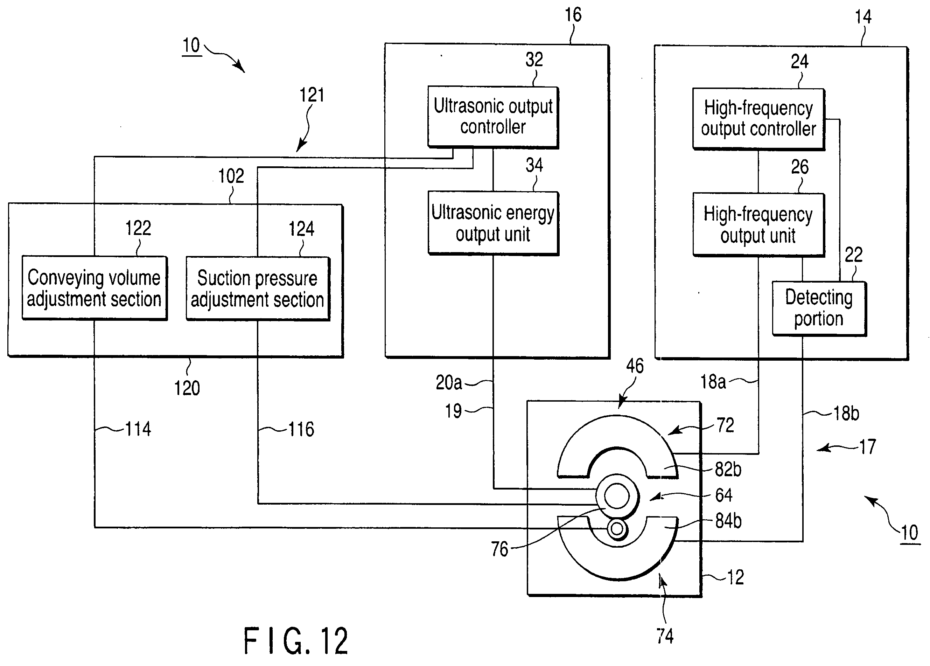

As shown in FIG. 11 and FIG. 12, the ultrasonic probe 76 (see FIG. 1A to FIG. 3D) which can be provided between first and second holding members 72, 74 is removed, and a cylindrical probe (hereinafter referred to as an ultrasonic suction probe) 176 is provided instead which can transmit ultrasonic vibrations and which can suck, for example, removed living tissues through its internal portion (suction passage 176a).

A medical treatment apparatus 10 includes an energy treatment instrument (treatment instrument) 12 called a handpiece, and a high-frequency energy source 14, an ultrasonic energy source 16, and a fluid feeding/suction unit 102.

The fluid feeding/suction unit 102 includes a bag 112 containing a physiological saline, a conveying tube (fluid feeding tube) 114, a suction tube 116, a suction tank 118 and a conveying volume/suction pressure adjuster 120. The conveying volume/suction pressure adjuster 120 has a conveying volume adjustment section 122 and a suction pressure adjustment section 124. The conveying volume/suction pressure adjuster 120 is detachably connected to the ultrasonic energy source 16 by a cable 121 and a connector 121a provided at its end.

In addition, the conveying tube 114 and the suction tube 116 are preferably formed of a chemical-resistant and flexible resin material such as PTFE.

The rear end of the conveying tube 114 is connected to the bag 112 containing the physiological saline, and provided side by side with the ultrasonic suction probe 176. The suction tube 116 is connected to the proximal end of the ultrasonic suction probe 176 and to the suction tank 118 for collecting, for example, sucked living tissues. That is, the energy treatment instrument 12 is provided with the conveying tube 114 and the suction tube 116. The conveying tube 114 is connected to the physiological saline bag 112 through the conveying volume adjustment section 122. The suction tube 116 is connected to the suction tank 118 via the suction pressure adjustment section 124. The conveying volume adjustment section 122 changes the inside diameter of the conveying tube 114 to control the volume of the physiological saline fed from the bag 112. The suction pressure adjustment section 124 adjusts the pressure for sucking, for example, living tissues into the suction tank 118.

An ultrasonic output controller 32 of the ultrasonic energy source 16 is connected to the fluid feeding/suction unit 102 located outside the ultrasonic energy source 16, that is, connected to the conveying volume adjustment section 122 and the suction pressure adjustment section 124.

The physiological saline bag 112 retains the physiological saline. The physiological saline in the bag 112 is fed to a living tissue (treatment portion) through the conveying tube 114 provided in the conveying volume adjustment section 122 by the activation of, for example, an unshown rotary pump. On the other hand, the living tissue is retained in the suction tank 118 by an unshown suction device through the suction passage 176a of the ultrasonic suction probe 176 and the suction tube 116 provided for the suction pressure adjustment section 124.

An ultrasonic transducer 43 is stored in a handle 42, and the ultrasonic suction probe 176 for transmitting the vibrations of the ultrasonic transducer 43 to the living tissue is stored in a shaft 44. The suction passage 176a is formed in the ultrasonic suction probe 176 over the entire length of the ultrasonic suction probe 176 to ultrasonically treat the living tissue and to suck the ultrasonically treated living tissue. It is preferable that the side surface of the ultrasonic suction probe 176 be curved or uneven. It is also preferable that the ultrasonic suction probe 176 have a structure for lateral vibrations or torsional vibrations.

Furthermore, the conveying tube 114 is provided side by side with the ultrasonic suction probe 176 of a sheath 54. Thus, the conveying tube 114 can pass the physiological saline sent from the physiological saline bag 112.

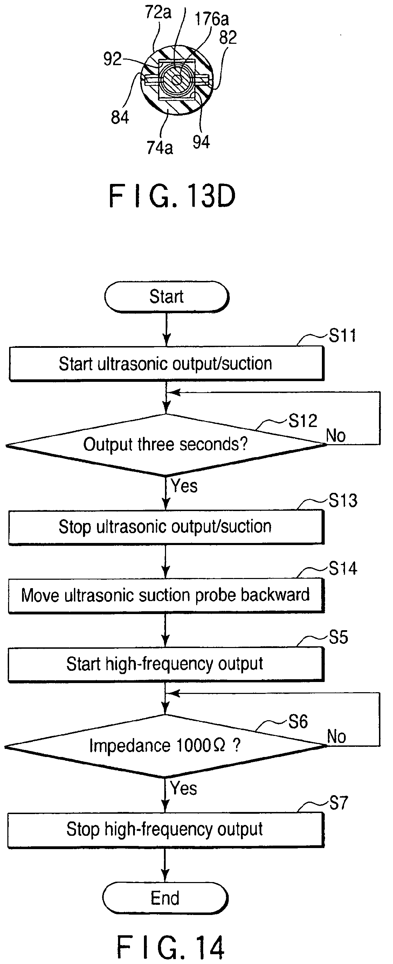

Now, the effects of the medical treatment apparatus 10 according to this embodiment are described along with a flowchart shown in FIG. 14.

This embodiment is similar in effects to the first embodiment expect that the fluid feeding/suction unit 102 is added.

Target living tissues are brought into contact with both of the first and second holding members 72, 74, and the ultrasonic suction probe 176 is disposed between the joint surfaces, in which state a foot switch or hand switch connected to the ultrasonic energy source 16 is operated. The vibrations of the ultrasonic suction probe 176 disposed between the first and second holding members 72, 74 are caused by the ultrasonic energy source 16 through a cable 19 and the ultrasonic transducer 43. At the same time, the physiological saline is fed to the living tissues (treatment portions), and the suction of the living tissue is started (S11). Cells in the surfaces of the living tissues are removed by cavitation caused to the living tissues due to the transmission of the ultrasonic vibrations, and collagens that contribute most to the joining of the living tissues are exposed on the joint surfaces. In this case, the physiological saline is fed to the living tissues being ultrasonically treated, so that the cells in the surfaces of the living tissues are sucked into the suction passage 176a of the ultrasonic suction probe 176 together with the physiological saline. Thus, collagens are exposed out of the target living tissues.

The ultrasonic treatment and suction of the living tissues are performed for a given period of time (e.g., three seconds) (S12), and are automatically stopped thereafter (S13). In addition, the feeding of the physiological saline to the treatment portions and the suction of the tissues are also stopped simultaneously with the stopping of the ultrasonic output.

Then, the ultrasonic suction probe 176 provided between the holding members 72, 74 is moved backward (S14). At the same time, the collagens exposed on the joint surfaces of the living tissues by the ultrasonic treatment are brought into close contact with each other by the pressing of electrodes 82b, 84b due to elastic members 92a, 94a, as described in the first embodiment.

Subsequently, a foot switch or hand switch connected to the high-frequency energy source 14 is operated. Then, energy is supplied to the first and second high-frequency electrodes 82b, 84b and the living tissues held between the first and second high-frequency electrodes 82b, 84b are denatured and dehydrated (S5 to S7).

As described above, the following can be said according to this embodiment.

In this embodiment, not only the living tissues are crushed by cavitation as in the first embodiment but also the crushed living tissues are sucked, such that the cells in the surfaces of the living tissues to be joined together can be effectively removed from the joint surfaces of the living tissues. Thus, collagens in the joint surfaces can be exposed.

As the fluid retained in the bag 112, it is preferable to use a fluid capable of inducing electric energy, such as an ionized conductive fluid permeable to living tissues. Such a fluid used includes, for example, a physiological saline, a hypertonic saline, a hypotonic saline or an electrolyte fluid replacement drug. The use of a highly viscous gel (fluid) such as hyaluronic acid is also permitted.

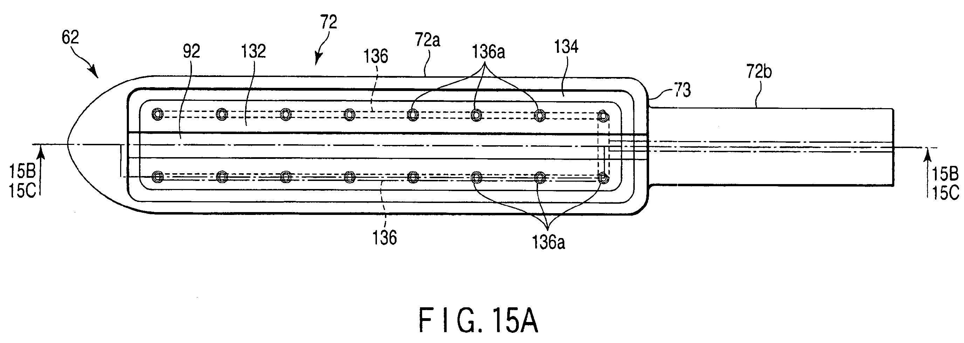

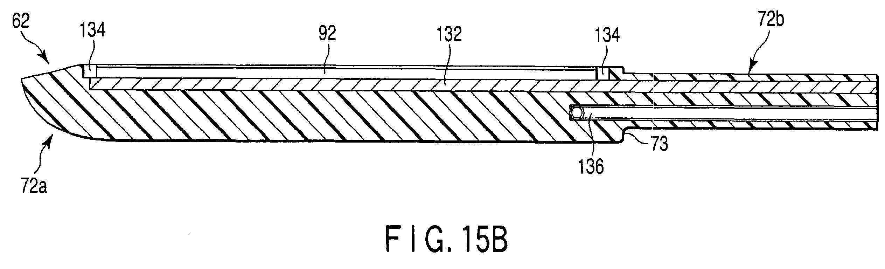

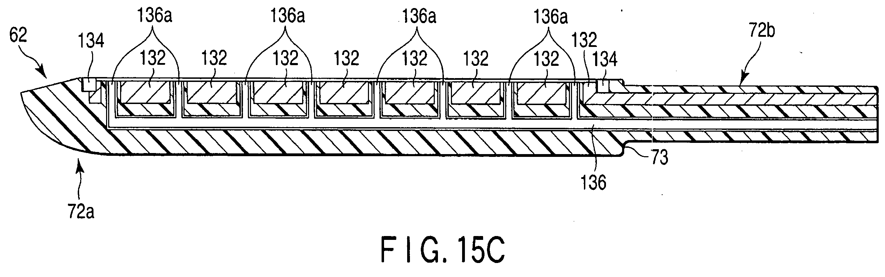

Moreover, as shown in FIG. 15A to FIG. 15C, apertures (conveyance apertures) 136a can be arranged in a holding surface 82 of the first holding member 72.

As shown in FIG. 15A to FIG. 15C, the first and second holding members 72, 74 have structures (conveyance mechanisms) capable of discharging the fluid to the target living tissues. A main body 72a of the first holding member 72 is provided with an electrode 132 having a planar surface to be in contact with living tissues. The electrode 132 is substantially rectangular, and an annular groove 134 serving as the passage of vapor generated from the living tissues is formed on the outer periphery of the electrode 132. On the other hand, a groove 92 where the ultrasonic probe 76 or the ultrasonic suction probe 176 is disposed is formed on the central axis of the main body 72a of the first holding member 72, as described in the first embodiment.

A conduit 136 is provided within the main body 72a of the first holding member 72. The conduit 136 bends substantially in the shape of L within the main body 72a of the first holding member 72, and a plurality of apertures (through-holes) 136a are formed in the conduit 136. The plurality of apertures 136a are open in the surface of the electrode 132. That is, the conduit 136 is in communication with the outside of the electrode 132. In particular, the plurality of apertures 136a open in the electrode 132 are formed with the same diameter at predetermined intervals at positions predetermined distance away from the central axis of the main body 72a of the first holding member 72. Thus, when a fluid such as a physiological saline runs through the conduit 136, the fluid is discharged from the apertures 136a of the conduit 136.

In addition, the apertures 136a are provided at equal intervals at positions substantially parallel with the groove 92 formed on the central axis of the main body 72a of the first holding member 72. The outer peripheries of the apertures 136a are covered with insulating materials. It is also preferable that the conduit 136 itself be formed of an insulating material. The conduit 136 is preferred to be, for example, a circularly cylindrical or squarely cylindrical, but is permitted to have various cross sectional shapes such as elliptically cylindrical or polygonally cylindrical shapes.

The conduit 136 is formed continuously to the handle 42 through a cylindrical member 52 of the shaft 44 or between the cylindrical member 52 and a sheath 54. The conduit 136 is in communication with the conveying tube 114, and enables the physiological saline fed from the bag 112 through the conveying tube 114 to be discharged from the apertures 136a provided inside the electrodes 132, 142 of the first and second holding members 72, 74.

The proximal end of the electrode 132 opposite to the side facing the second holding member 74 is connected to a cable 17 extending from the handle 42 via a first conducting line 18a.

Although not shown, the second holding member 74 is formed symmetrically to the first holding member 72. Here, for convenience of explanation, the numeral 142 is assigned to the electrode provided in the second holding member 74, the numeral 144 is assigned to an annular groove, the numeral 146 is assigned to a conduit, and the numeral 146a is assigned to apertures.

In the case where the electrodes 132, 142 of the first and second holding members 72, 74 have the same potential (homopolar), living tissues in contact with the electrode 132 of the first holding member 72 and the electrode 142 of the second holding member 74 are heated when supplied with energy (high-frequency electric power) from the high-frequency energy source 14. In this case, the electrodes 132, 142 serve as sensors to measure a current, a voltage, etc. flowing the electrodes 132, 142 through the living tissues. Then, the electrodes 132, 142 input relevant signals to a detecting portion 22 of the high-frequency energy source 14 through the first and second conducting lines 18a, 18b.

In addition, the conduits 136, 146 of the first and second holding members 72, 74 extend to the handle 42 through the cylindrical member 52 of the shaft 44. These conduits 136, 146 extend from the handle 42 as tubes (not shown) provided side by side with the first and second conducting lines 18a, 18b, and are connected to, for example, the conveying tube 114 (see FIG. 11). Thus, a liquid such as a conductive fluid can be injected into the apertures 136a, 146a through the conduits 136, 146.

The apertures 136a, 146a are circular in FIG. 15A to FIG. 15C, but are not limited to the circular shape and are permitted to have various shapes such as elliptic and polygonal shapes. Further, the apertures 136a in the first holding member 72 and the apertures 146a in the second holding member 74 are not exclusively aligned at predetermined intervals along the longitudinal direction of the first and second high-frequency electrodes 132, 142, and are permitted to be arranged in a plurality of lines or at random.

In the holding surface 82 of the first holding member 72 shown in FIG. 15A to FIG. 15C, both conveyance apertures for conveying the fluid and suction apertures for sucking can be arranged by, for example, dividing the conduit 136 into two parts. That is, a conveyance mechanism and a suction mechanism can be provided side by side in the first holding member 72. Moreover, although not described in detail, the conveyance apertures 136a in the holding surface 82 can be replaced with suction apertures and thus changed to the suction mechanism.

### [Third Embodiment]

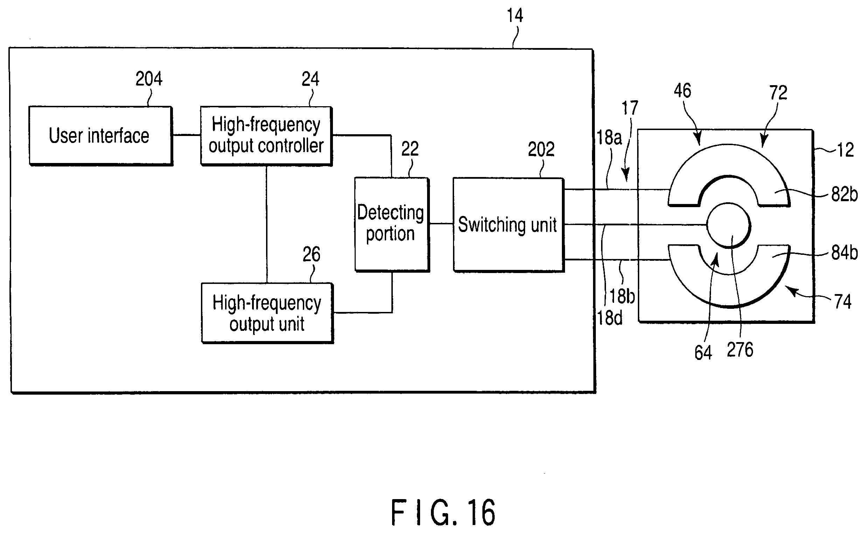

Next, a third embodiment is described with FIG. 16 to FIG. 19B. This embodiment is a modification of the first embodiment, and the same parts as the parts described in the first embodiment are provided with the same numerals and are not described in detail.

As shown in FIG. 16 to FIG. 17D, the ultrasonic probe 76 which can be provided between first and second holding members 72, 74 is removed, and a rod high-frequency electrode (energy emitter) 276 is provided instead.

As shown in FIG. 16, a high-frequency energy source 14 of a medical treatment apparatus 10 includes a detecting portion 22, a high-frequency output controller 24, a high-frequency output unit 26, a switching unit 202 and a user interface 204.

The switching unit 202 is connected to the detecting portion 22 and to an energy treatment instrument 12. The switching unit 202 closes/opens a circuit between the first electrode 82b, the second electrode 84b and the rod electrode 276, and the energy treatment instrument 12, in order to change the flow of a current. In the present embodiment, the circuit is switched between a first stage output and a second stage output that will be described later.

In addition, the user interface 204 is used, for example, to indicate the current state of the high-frequency energy source 14 or to set a threshold value for living tissues between the frequency electrodes 82b, 84b, 276.

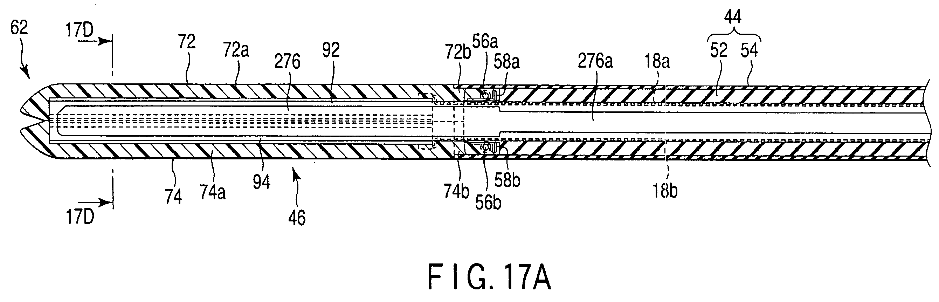

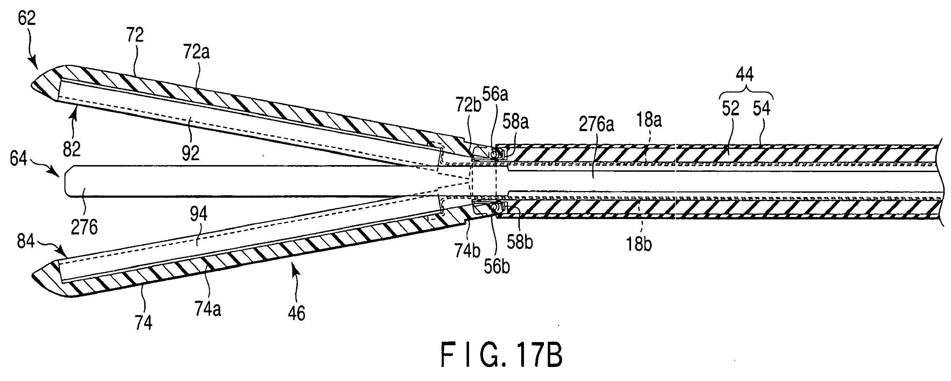

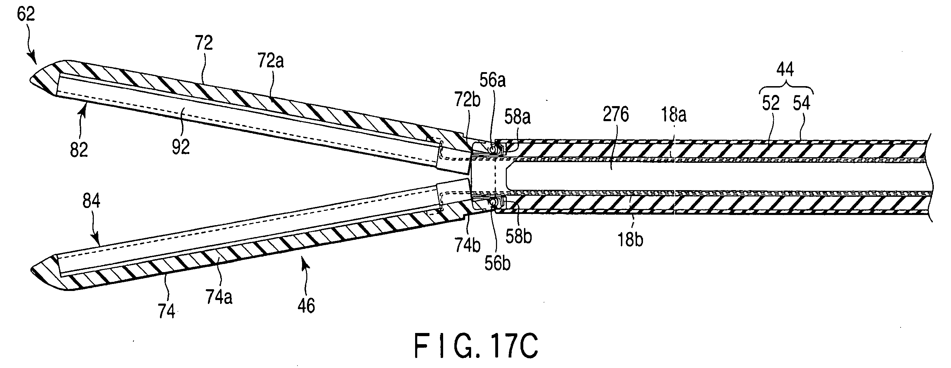

A second knob 42b of a handle 42 is connected to the proximal end of a lengthwise feed rod 276a shown in FIG. 17A to FIG. 17C. The distal end of the lengthwise feed rod 276a is connected to the proximal end of the rod electrode 276 through a shaft 44. That is, instead of the ultrasonic probe 76, the rod electrode 276 is disposed between the first and second holding members 72, 74. If the second knob 42b provided in the handle 42 is moved toward the operator, the rod electrode 276 disposed between the holding members 72, 74 of the energy treatment instrument 12 shown in FIG. 17A to FIG. 17C axially moves to the side proximate to the operator. Then, as shown in FIG. 17C, the distal end of the rod electrode 276 is stored in the distal end of a cylindrical member 52 of the shaft 44. If the second knob 42b is moved to the side of the holding members 72, 74, that is, distally with respect to the operator, the distal end of the rod electrode 276 comes out of the distal end of the cylindrical member 52 of the shaft 44 and is again located between the holding members 72, 74 as shown in FIG. 17B.

Inside the handle 42, there are provided a first conducting line 18a which supplies a high-frequency current to the electrode 82b provided in the first holding member 72, a second conducting line 18b which supplies a high-frequency current to the electrode 84b of the second holding member 74, and a third conducting line 18d which supplies a high-frequency current to the rod electrode 276. The first to third conducting lines 18a, 18b, 18d are provided in a cable 17. That is, the third conducting line 18d is electrically connected to the rod electrode 276. Thus, the first to third conducting lines 18a, 18b, 18d are connected to a connector 17a of the cable 17.

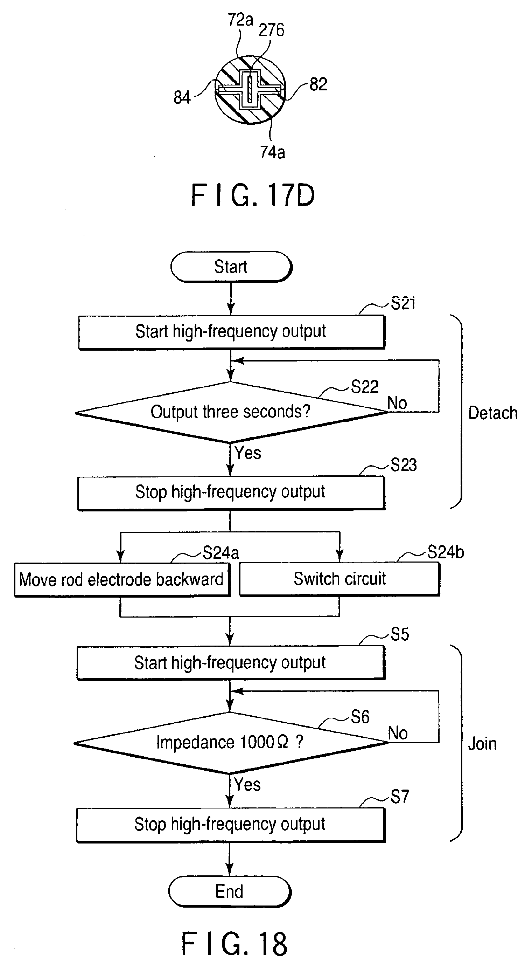

While the sectional shape of the rod electrode 276 is, for example, rectangular as shown in FIG. 17D, its cross section is permitted to have various shapes such as circular and polygonal shapes. The sectional shape of grooves 92, 94 of holding surfaces 82, 84 is preferred to be similar to the sectional shape of the rod electrode 276, but is permitted to be various shapes such as elliptic and polygonal shapes.

The rod electrode 276 is smaller in surface area than the first and second electrodes 82b, 84b of the first and second holding members 72, 74. Thus, when the first and second electrodes 82b, 84b are homopolar and high-frequency energy is output to the living tissues between the electrodes 82b, 84b and the rod electrode 276, the current density increases on the surface of the rod electrode 276, so that the living tissues can transpire.

Furthermore, in order to sufficiently treat the living tissues held between main bodies 72a, 74a of the first and second holding members 72, 74, it is preferable that the electrodes 82b, 84b be continuously formed in a direction perpendicular to the longitudinal direction of main bodies 72a, 74a, as shown in FIG. 17A to FIG. 17D (FIG. 17D in particular). The use of such electrodes 82b, 84b makes it possible to have a greater contact area between the electrodes 82b, 84b and the living tissues. Thus, pressure sufficient to treat the living tissues can be applied without disposing elastic members 92a, 94a (see FIG. 3A to FIG. 3C).

Now, the effects of the medical treatment apparatus 10 according to this embodiment are described.

Here, during the above-mentioned first stage output, the first electrode 82b and the second electrode 84b have the same polarity, and the rod electrode 276 has a polarity different from the polarity of the first electrode 82b and the second electrode 84b. Thus, in the first stage output, the current flows from the first and second electrodes 82b, 84b to the rod electrode 276 under the control of the switching unit 202.

Before the second stage output, the rod electrode 276 is stored in the shaft 44. Moreover, before the second stage output, the circuit is switched by the switching unit 202 so that the first electrode 82b and the second electrode 84b differ in polarity. As a result, a current flows through the living tissue between the first electrode 82b and the second electrode 84b during the second stage output.

The operation of the medical treatment apparatus 10 is described in detail below along with a flowchart shown in FIG. 18.

As described in the first embodiment, living tissues are held between the holding surfaces 82, 84 of the main bodies 72a, 74a of the first and second holding members 72, 74. At this point, the target living tissues are in contact with a contact surface 82a and the first high-frequency electrode 82b of the holding surface 82 of the first holding member 72 and with a contact surface 84a and the second high-frequency electrode 84b of the holding surface 84 of the second holding member 74. In this state, a foot switch or hand switch connected to the frequency energy source 14 is operated.

In this case, the switching unit 202 is set to the first stage output. Therefore, energy is supplied from the frequency energy source 14 to the first electrode 82b and the second electrode 84b which are homopolar and to the rod electrode 276 different in polarity from the electrodes 82b, 84b through the conducting lines 18a, 18b, 18d. As a result, a current flows from the first electrode 82b and the second electrode 84b to the rod electrode 276 through the living tissues (S21).

Here, the area of the living tissue in contact with the rod electrode 276 is smaller than the area of the living tissue in contact with the first electrode 82b and the second electrode 84b. Thus, current density in the tissue surface in contact with the rod electrode 276 is higher than current density in the first and second electrodes 82b, 84b. As a result, the living tissues around the rod electrode 276 can efficiently transpire. Thus, epithelial tissues are detached and removed, and layers containing collagen are exposed in the joint surfaces of the living tissues. It is judged whether a given period of time (e.g., three seconds) has passed since the start of the outputs from the first electrode 82b and the second electrode 84b which are homopolar and the output from the rod electrode 276 different in polarity from the electrodes 82b, 84b (S22). Thus, the high-frequency output is automatically stopped after three seconds have passed (S23).

In addition, simultaneously with the start of the first stage output, the impedance Z of the living tissues in contact with the rod electrode 276 can be detected by the detecting portion 22 in the frequency energy source 14. The impedance Z at the beginning of the first stage output (initial value) is, for example, about 50[Ω], which however varies depending on the size and shape of the electrodes 82b, 84b, 276. Then, as high-frequency electric power is applied to the living tissues and the living tissues are cauterized, the value of the impedance Z once drops from about 50[Ω], and then rises. The first stage output may be stopped when the impedance Z of the living tissues has increased to, for example, about 1000[Ω] rather than when a given period of time (e.g., three seconds) has passed since the start till the end of the high-frequency output.

After the end of the application of electricity to the living tissues between the first and second electrodes 82b, 84b and the rod electrode 276 different in polarity from the electrodes 82b, 84b, an indication saying, for example, "rod electrode can be moved backward" is displayed on the user interface 204 of the frequency energy source 14. This indication represents that the first stage output has been finished. After confirming this indication, the operator (user) releases the foot switch or hand switch. It is also preferable that "first stage output finished" be displayed on the user interface 204.

After the frequency energy source 14 has stopped the output of the high-frequency energy from the high-frequency output unit 26, the switching unit 202 automatically switches the circuit to the second stage output (S24b). On the other hand, simultaneously with the switch of the circuit by the switching unit 202 or at a proper period (before or after the switch of the circuit), the rod electrode 276 is moved backward with the first and second holding members 72, 74 closed, and is stored in the shaft 44 (S24a). At the same time, the second knob 42b provided in the handle 42 is moved toward the operator. Then, the rod electrode 276 located between the holding members 72, 74 of the energy treatment instrument 12 axially moves toward the operator through the shaft 44.

In the second stage output, the circuit is switched so that the first electrode 82b and the second electrode 84b differ in polarity. The foot switch or hand switch connected to the frequency energy source 14 is again operated. As a result, a current flows through the living tissue between the first and second electrodes 82b, 84b during the second stage output. That is, a high-frequency current is applied to the first high-frequency electrode 82b and the second high-frequency electrode 84b via the target living tissues (S5). Thus, the target living tissues between the first high-frequency electrode 82b and the second high-frequency electrode 84b is heated.

Simultaneously with the start of the second stage output, the impedance Z of the living tissues in contact with the first and second electrodes 82b, 84b is detected by the detecting portion 22 in the frequency energy source 14. The impedance Z at the beginning of the treatment (initial value) is, for example, about 50[Ω], which however varies depending on the size and shape of the electrodes 82b, 84b. Then, as high-frequency electric power is applied to the living tissues and the living tissues are cauterized, the value of the impedance Z once drops from about 50[Ω], and then rises. Such a rise in the value of the impedance Z represents that the living tissues are losing water and drying. Consequently, as the target living tissues are heated and cauterized, the living tissues are gradually denatured and dehydrated and thus united.

Then, it is judged whether the calculated impedance Z has exceeded, for example, 1000[Ω] (not limited to this value and any value can be set) set as the threshold value in the high-frequency output controller 24 (S6). When the impedance Z is judged to have exceeded a threshold value of 1000[Ω], the high-frequency output controller 24 stops the output of the high-frequency electric power from the high-frequency output unit 26 (S7).

After the end of the application of electricity to the living tissues between the first high-frequency electrode 82b and the second high-frequency electrode 84b, an indication saying, for example, "treatment finished" is displayed on the user interface 204 of the frequency energy source 14. This indication represents that the second stage output has been finished. After confirming this indication, the operator (user) releases the foot switch or hand switch. It is also preferable that "second stage output finished" be displayed on the user interface 204.

As described above, the following can be said according to this embodiment.

In this embodiment, the high-frequency electrode (rod electrode) 276 is used instead of the ultrasonic probe 76 in the first embodiment to desquamate the living tissues on the joint surfaces. The high-frequency energy does not have such specific properties of only preserving collagen as in the treatment with the ultrasonic energy. However, in the case of, for example, epithelial tissues, collagen present deeper than the epithelial tissues can be exposed when the high-frequency energy is used to cause the transpiration of the cell components present on the surface.

Although the emission of the high-frequency energy is continued for a given period of time in the first stage output and the second stage output in the case described in the present embodiment, it is also advantageous to provide an idle period in the high-frequency output or to repeat lower outputs and high outputs. As to a termination condition for a treatment (termination condition for the second stage output), the treatment may be automatically ended not only judging whether the impedance Z has exceeded the threshold value set as the termination condition but also after the high-frequency energy is output for a certain period of time.

As in the first and second embodiments, it is also preferable that the elastic members 92a, 94a be disposed within the holding members 72, 74 so that the electrodes 82b, 84b in the grooves 92, 94 of the holding members 72, 74 may be pressed from the rear. As a result, holding pressure can be applied to the living tissues when the rod electrode 276 is not located between the holding members 72, 74.

Moreover, although the transpiration by the first stage output is achieved here by the application of a current across the rod electrode 276 and the first and second electrodes 82b, 84b, it is also preferable to perform a treatment using another electrode provided side by side with the rod electrode 276.

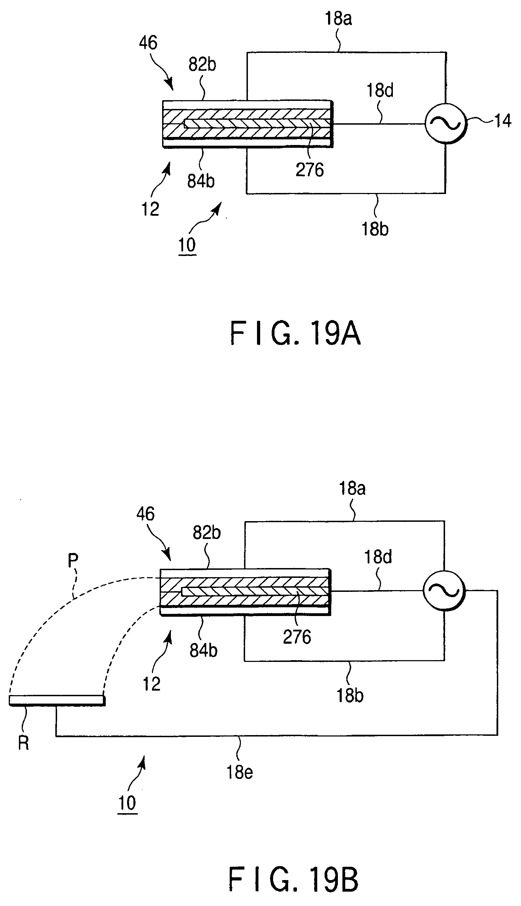

In addition, the bipolar treatment as schematically shown in FIG. 19A has been described above in the third embodiment. That is, in the case described, electricity is applied to the living tissues between the first and second electrodes 82b, 84b and the rod electrode 276 and to the living tissues between the first electrode 82b and the second electrode 84b.

Here, as shown in FIG. 19B, it is also preferable to perform a monopolar treatment. In this case, a return electrode plate R is attached to a patient P to be treated. The return electrode plate R is connected to the high-frequency energy source 14 via a conducting line 18e.

Then, when the first and second electrodes 82b, 84b are homopolar, electricity is applied to the return electrode plate R and the living tissue between the first and second electrodes 82b, 84b. In this case, the area of the living tissue in contact with the first and second electrodes 82b, 84b is sufficiently smaller than the area of the living tissue in contact with the return electrode plate R. Therefore, energy density is higher for the living tissue in contact with the first and second electrodes 82b, 84b. Thus, the living tissue held between the first and second electrodes 82b, 84b is treated.

It is also possible to apply electricity to the living tissue between the rod electrode 276 and the return electrode plate R. In this case, the area of the living tissue in contact with the rod electrode 276 is sufficiently smaller than the area of the living tissue in contact with the return electrode plate R. Therefore, energy density is higher for the living tissue in contact with the rod electrode 276. Thus, the joint surface of the living tissue in contact with the rod electrode 276 (here, the living tissue held between the first and second holding members 72, 74) is treated.

### [Fourth Embodiment]

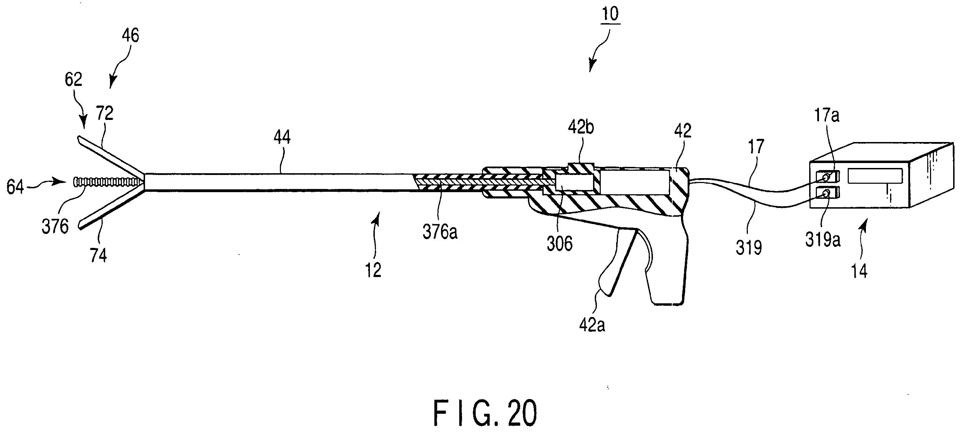

Next, a fourth embodiment is described with FIG. 20 to FIG. 23. This embodiment is a modification of the first embodiment, and the same parts as the parts described in the first embodiment are provided with the same numerals and are not described in detail.

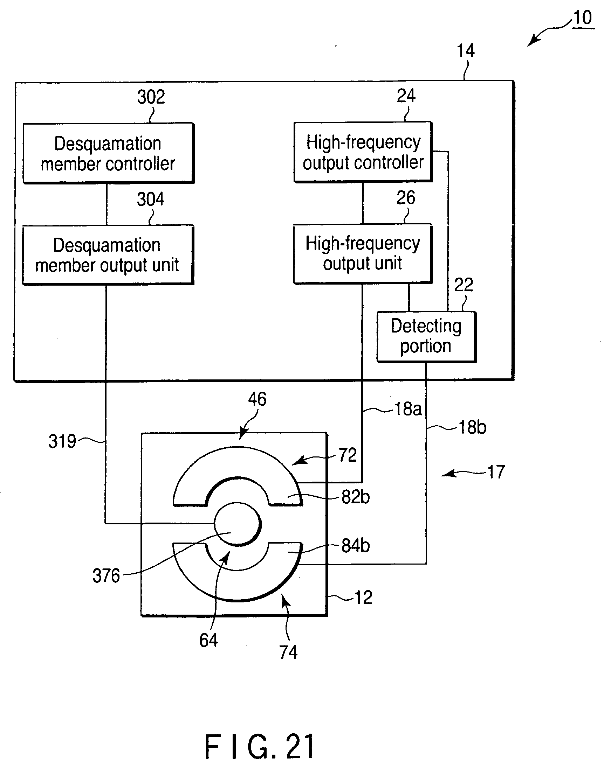

As shown in FIG. 20 and FIG. 21, a medical treatment apparatus 10 includes an energy treatment instrument (medical treatment instrument) 12, and a high-frequency energy source 14 for providing high-frequency energy to the energy treatment instrument 12.

As shown in FIG. 21, the high-frequency energy source 14 includes a detecting portion 22, a high-frequency output controller 24, a high-frequency output unit 26, a desquamation member controller 302 and a desquamation member output unit 304. The desquamation member output unit 304 for driving a desquamation member moving mechanism 306 of a mechanical desquamation member 376 is connected to the desquamation member controller 302.

As shown in FIG. 20, in a handle 42, the mechanical desquamation member moving mechanism 306 such as a linear motor is provided to rotate or vibrate the desquamation member 376 disposed between holding members 72, 74. In order to acquire electric power, the desquamation member moving mechanism 306 is connected to the high-frequency energy source 14 by a connector 319a of a cable 319 extending from the energy treatment instrument 12. The mechanical desquamation member moving mechanism 306 is connected to the proximal end of a lengthwise feed rod 376a inserted through a cylindrical member 52 of a shaft 44. The distal end of the lengthwise feed rod 376a is formed integrally with the proximal end of the desquamation member 376 disposed between the holding members 72, 74.

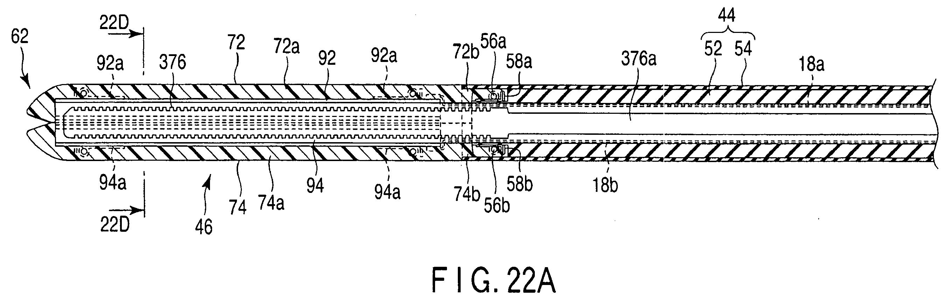

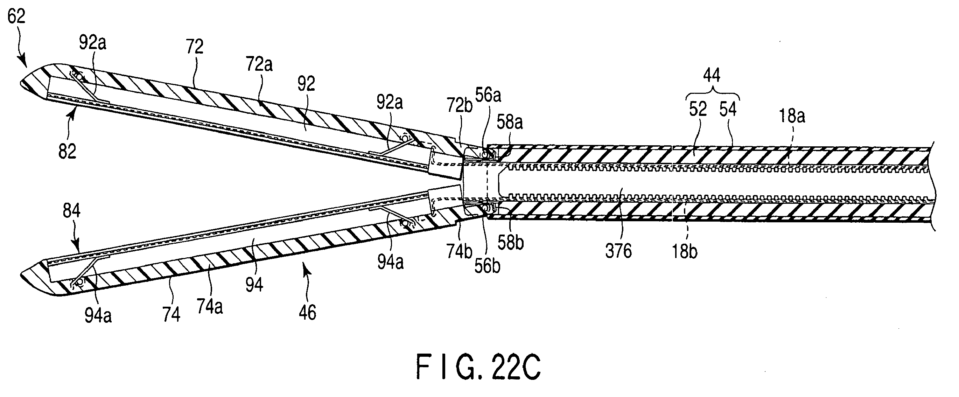

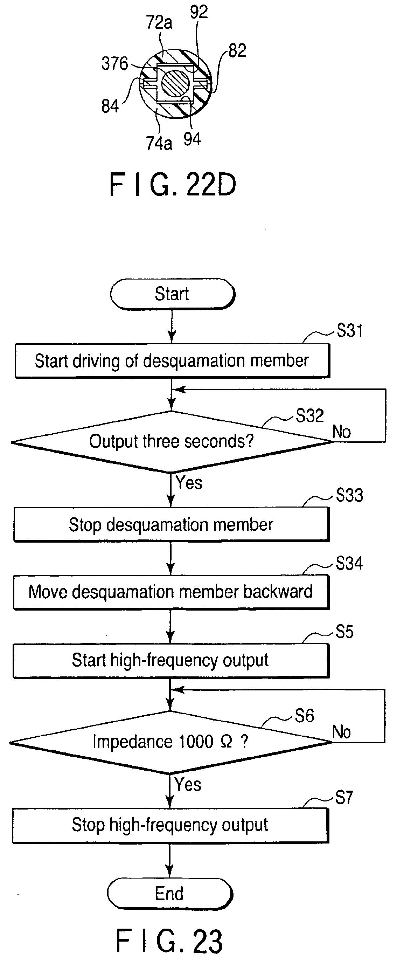

As shown in FIG. 22D, the cross section of the desquamation member 376 is, for example, circular, but is permitted to have various shapes such as an elliptic and polygonal shape. The sectional shape of grooves 92, 94 of main bodies 72a, 74a of the holding members 72, 74 is preferred to be similar to the outer shape (circular shape) of the desquamation member 376, but is permitted to be various shapes such as elliptic and polygonal shapes. The use of a plurality of desquamation members 376 is also permitted.

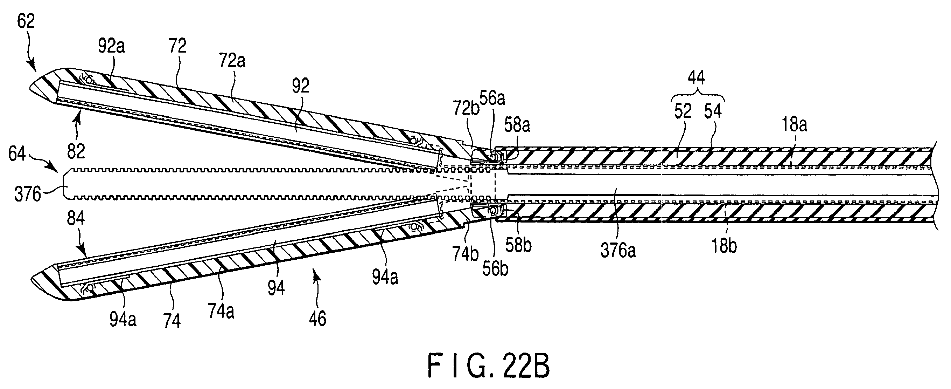

As shown in FIG. 22B, the surface of the desquamation member 376 has uneven portions such as axial or annular grooves so that the surface layer of the living tissue may be easily desquamated. The edges of the uneven portions are preferred to be sharp.

Now, the effects of the medical treatment apparatus 10 according to this embodiment are described along with a flowchart shown in FIG. 23.

Living tissues are held between the first and second holding members 72, 74, and the desquamation member 376 is disposed between the living tissues to be joined together. Then, a hand switch or foot switch connected to the frequency energy source 14 is pressed.

The detachment member moving mechanism 306 inside the handle 42 is rotated or vibrated by the frequency energy source 14 via the cable 319. Thus, the desquamation member moving mechanism 306 transmits the rotational or vibrational movement to the lengthwise feed rod 376a. In addition, the vibrations referred to here mean vibrations lower in frequency than ultrasonic vibrations. The lengthwise feed rod 376a transmits its rotational or vibrational movement to the detachment member 376. This rotates or vibrates the desquamation member 376 disposed between the holding members 72, 74. Components of the surfaces of the living tissues are desquamated or cut by the rotation or vibrations of the desquamation member 376, and layers containing collagen having relatively high strength are exposed on the joint surfaces (S31).

In addition, the detaching operation by the desquamation member 376 is performed for a given period of time (S32). After a given period of time from the start of the output, for example, three seconds, the detachment member controller 302 stops the output of electric power from the physical desquamation member output unit 304. Thus, the rotation or vibration by the desquamation member moving mechanism 306 is also stopped. Accordingly, the rotation or vibration by the desquamation member 376 is also stopped (S33).

Then, the desquamation member 376 between the first and second holding members 72, 74 is moved backward. When a second knob 42b of the handle 42 is moved toward the operator, the desquamation member 376 located between the holding members 72, 74 of the energy treatment instrument 12 axially moves toward the operator through the shaft 44, and the distal end of the desquamation member 376 is stored in the distal end of the shaft 44 (S34).

While details have been described in the first embodiment and are therefore omitted, the frequency energy source 14 then drives the high-frequency output unit 26 therein under the control of the high-frequency output controller 24, and outputs high-frequency electric power from the high-frequency output unit 26 (S5). As a result, the living tissues are joined together (S6, S7).

As in the first embodiment, when the impedance Z detected by the detecting portion 22 is judged to have exceeded a threshold value of 1000[Ω], the high-frequency output controller 24 stops the output of the high-frequency electric power from the high-frequency output unit 26.

As described above, the following can be said according to this embodiment.

In this embodiment, in order to desquamate the living tissues in the joint surfaces, the desquamation member 376 for providing a mechanical stimulus such as friction is used instead of the ultrasonic probe 76 in the first embodiment. The mechanical stimulus does not have such specific properties of only preserving collagen as in the treatment with the ultrasonic energy. However, if, for example, cell components such as epitheliums present on the surface are removed by the friction, collagen present in deeper parts can be exposed.

The invention is set out in the appended claims that follow.

## Claims

1. A treatment instrument (10) configured to join target living tissues, comprising: a shaft (44) having a longitudinal axis;
a holding portion disposed at the distal end of the shaft (44), and including a pair of holding members (72, 74) having holding surfaces (82,84) configured to open and close in order to hold the target living tissues; the holding portion further including
an energy emitter (82b, 84b) provided on the holding surfaces (82,84) of the holding members (72,74) and configured to emit energy and to generate heat within the target living tissues held by the holding portion to join said tissues; a treating portion (64) comprising an ultrasonic probe (76); wherein the treating portion (64) is configured to move along the longitudinal axis when the holding members (72,74) are closed, from a first position within the shaft (44) to an extended position between the holding members (72,74); and wherein the ultrasonic probe (76) is configured to transmit ultrasonic vibrations to remove surface tissues of joint surfaces of the target living tissues and expose protein including collagen in a submucosa.

2. The treatment instrument according to claim 1, wherein the energy emitter (82b, 84b) is configured to emit the energy and to generate the heat after the surface tissues of the joint surfaces of the target living tissues are removed and transmission of the ultrasonic vibration to the ultrasonic probe (76) is stopped.

## Patentansprüche

1. Behandlungsgerät (10), das dazu eingerichtet ist, lebende Zielgewebe zu verbinden, umfassend:
eine Welle (44) mit einer Längsachse;
einen Halteabschnitt, der an dem distalen Ende der Welle (44) angeordnet ist und ein Paar von Halteelementen (72, 74) umfasst, welche Halteoberflächen (62, 64) haben, die dazu eingerichtet sind, sich zu öffnen und zu schließen, um die lebenden Zielgewebe zu halten; wobei der Halteabschnitt ferner umfasst
einen Energieemitter (82b, 84b), der an den Halteoberflächen (82, 84) der Halteelemente (72, 74) vorgesehen und dazu eingerichtet ist, Energie zu emittieren und Wärme in den von dem Halteabschnitt gehaltenen lebenden Zielgeweben zu erzeugen, um die Gewebe zu verbinden;
einen Behandlungsabschnitt (64), der eine Ultraschallsonde (76) umfasst, wobei der Behandlungsabschnitt (64) dazu eingerichtet ist, sich, wenn die Halteelemente (72, 74) geschlossen sind, entlang der Längsachse von einer ersten Position innerhalb der Welle (44) zu einer verlängerten Position zwischen den Halteelementen (72, 74) zu bewegen, und wobei die Ultraschallsonde (76) dazu eingerichtet ist, Ultraschallschwingungen zu übertragen, um Oberflächengewebe von Verbindungsoberflächen der lebenden Zielgewebe zu entfernen und Kollagen enthaltendes Protein in einer Submucosa freizulegen.

2. Behandlungsinstrument gemäß Anspruch 1, wobei der Energieemitter (82b, 84b) dazu eingerichtet ist, die Energie zu emittieren und die Wärme zu erzeugen, nachdem die Oberflächengewebe der Verbindungsoberflächen der lebenden Zielgewebe entfernt worden sind und die Übertragung der Ultraschallschwingung an die Ultraschallsonde (76) gestoppt worden ist.

## Revendications

1. Instrument de traitement (10) configuré pour relier des tissus vivants cible, comprenant :
un arbre (44) comportant un axe longitudinal ;
une partie de maintien disposée au niveau de l'extrémité distale de l'arbre (44), et comprenant une paire d'éléments de maintien (72, 74) comportant des surfaces de maintien (82, 84) configurée pour s'ouvrir et se fermer afin de maintenir les tissus vivants cible ;
la partie de maintien comprenant en outre
un émetteur d'énergie (82b, 84b) prévu sur les surfaces de maintien (82, 84) des éléments de maintien (72, 74) et configuré pour émettre de l'énergie et pour générer de la chaleur à l'intérieur des tissus vivants cible maintenus par la partie de maintien pour relier lesdits tissus ;
une partie de traitement (64) comprenant une sonde à ultrasons (76) ; dans lequel la partie de traitement (64) est configurée pour se déplacer le long de l'axe longitudinal lorsque les éléments de maintien (72, 74) sont fermés, d'une première position à l'intérieur de l'arbre (44) à une position étendue entre les éléments de maintien (72, 74) ; et
dans lequel la sonde à ultrasons (76) est configurée pour transmettre des vibrations ultrasonores pour enlever des tissus de surface des surfaces de liaison des tissus vivants cible et exposer les protéines incluant le collagène dans une sous-muqueuse.

2. Instrument de traitement selon la revendication 1, dans lequel l'émetteur d'énergie (82b, 84b) est configuré pour émettre l'énergie et pour générer de la chaleur après que les tissus de surface des surfaces de liaison des tissus vivants cible ont été enlevés et que la transmission des vibrations ultrasonores de la sonde à ultrasons (76) a été arrêtée.
